# EUROPEAN PATENT APPLICATION

(11) **EP 4 809 908 A1**
(43) Date of publication of application: **23.09.2026**
(21) Application number: 25382275.3
(22) Date of filing: 21.03.2025
(51) Int. Cl.: A61B 5/00, A61B 8/08, A61B 8/00, A61B 5/01, A61B 17/00

(54) **DEVICE AND METHOD FOR OPTOACOUSTIC MEASUREMENT OF HEAT DEPOSITION IN NON-THERAPEUTIC PROCEDURES**

(71) Applicant: Universidad Autónoma de Madrid, 28049 Madrid (ES)
(72) Inventor: AGUIRRE BUENO, Juan, 28049 Madrid (ES); CONTADOR PACHÓN, Sergio, 28049 Madrid (ES)
(74) Representative: Hoffmann Eitle

(57) **Abstract**

The present invention discloses a device and a method for non-therapeutical optoacoustic measurement of deposited heat in one or more target layers within human or animal skin tissue. The device comprises a radiation source configured to emit pulsed electromagnetic radiation suitable for generating optoacoustic waves in the skin tissue upon interaction with the target layers. The device further comprises an ultrasonic detector, comprising a transducer with a sensing surface, suitable for detecting the optoacoustic waves produced by the interaction of the emitted radiation with the target layers, and a processing and/or controlling unit, in communication a memory and with both the radiation source and the ultrasonic detector, wherein the memory comprises instructions for analysing the detected signals. The memory further comprises processor readable instructions for determining the distance between the transducer's sensing surface and the target layers based on the time of radiation emission and receival of the optoacoustic signals, and, optionally for calculating the relative distance and thickness of multiple target layers. The device is further capable of generating data and/or images that provide information on the deposited heat in the target layers, their distance from the transducer, and/or their respective thicknesses. It is noted that the target layers comprise at least an ink layer and/or a melanin layer.

## Description

### Technical field of the invention

The present invention belongs to the field of non-therapeutic thermo-mechanical lysis treatments, specifically to tattoo or skin spot removal systems and methods. More particularly, it relates to a device and method for optimising the parameters of tattoo removal using thermo-mechanical lysis, based on optoacoustic measurements that assess the heat deposition in target layers of the skin. This invention utilises a combination of pulsed laser irradiation and ultrasound detection to predict and enhance the effectiveness of tattoo removal treatments.

### Background of the invention

Tattoo removal procedures have gained widespread use as individuals seek effective ways to eliminate unwanted tattoos. The most common method relies on thermo-mechanical lysis, a laser-based process that fragments tattoo ink particles by delivering short, high-energy pulses of light. The principle behind this technique is that tattoo pigments absorb laser energy more effectively than the surrounding skin. Light absorption leads to heat deposition in the tissue, which, in turn leads to the thermal and mechanical decomposition of ink granules. Once fragmented, these particles are gradually cleared away by the body's immune system over the course of several weeks or months.

The same principle is also applied in cosmetic dermatology for skin treatments such as depigmentation procedures aimed at whitening the skin or removing hyperpigmented lesions. Conditions such as melasma, post-inflammatory hyperpigmentation, and lentigines (age spots) are commonly treated with laser therapy by selectively targeting the melanin-rich areas. In these procedures, controlled laser energy is used to break down excess melanin deposits in the skin, promoting a more uniform complexion. However, the effectiveness and safety of such treatments depend on precise control of laser parameters to avoid excessive thermal damage to surrounding tissues.

Despite its widespread use, thermo-mechanical lysis has several limitations that significantly affect treatment efficacy, predictability, and safety. Currently, dermatologists and laser practitioners rely on visual assessment and trial-and-error to determine laser parameters. The first-pass reaction of the skin-typically characterized by temporary whitening due to sub-epidermal gas bubble formation-is used as an immediate treatment endpoint. However, this method provides limited real-time feedback, as the true effectiveness of the treatment is only evident weeks later, when the immune system has cleared the ink fragments or the skin's melanin distribution has stabilized.

Wavelength selection is currently largely empirical. Since tattoo pigments and melanin deposits absorb light differently, selecting the optimal wavelength for maximum absorption in the target area while avoiding excessive heat deposition in non-target tissues, such as the surrounding skin, is difficult. Visual inspection of tattoo colour or hyperpigmented skin lesions is an unreliable predictor of absorption characteristics, particularly for darker skin tones, where melanin interferes with light penetration and absorption, resulting in an unknown heat deposition spectrum

One of the most significant safety concerns is the risk of collateral skin damage, particularly in individuals with higher melanin concentrations. If laser energy is inadvertently absorbed by the melanin layer instead of the target tattoo ink or hyperpigmented spot, it can lead to burns, hypopigmentation, and scarring. This is particularly problematic when attempting to treat dark or deeply embedded tattoos or large pigmented lesions, where higher energy levels are required.

Laser spot size optimization is another critical factor that remains largely unaddressed. The ability to control and adjust the laser's impact area based on the characteristics of the tattoo or pigmented region could improve treatment uniformity and prevent excessive tissue damage.

Currently available laser treatment techniques do not provide real-time, quantitative feedback on energy absorption and heat deposition in the target layers versus the surrounding skin. As a result, practitioners lack an objective method to predict the likelihood of adverse effects such as skin whitening, burns, or insufficient pigment removal. Additionally, there is no widely adopted system that can offer accurate visual feedback on heat deposition in each target layer, ink fragmentation, tissue response, and optimal treatment parameters during the procedure.

There is, therefore, an emerging need for a device and method that can address these challenges by providing real-time, non-invasive measurements of heat deposition in tattoo ink, melanin-rich skin regions, and/or other target layers. Such a system would allow for precise laser parameter selection, risk minimization, and improved treatment outcome prediction across multiple non-therapeutic dermatological applications, including tattoo removal and skin-spot elimination, promoting thus the reduction of unwanted skin whitening and lesions.

### Summary of the invention

The present invention relates to a device and associated method for optimizing and predicting the outcome of non-therapeutical selective thermo-mechanical lysis interventions, particularly in tattoo removal, but also applicable to the removal of skin spots with high melanin concentrations and/or skin whitening. The invention overcomes the limitations of existing methods by providing a non-invasive, pre-treatment imaging tool that utilizes optoacoustic measurements to assess the deposition of heat within target layers below the skin surface. This information may thus be used in some embodiments for the precise selection of laser parameters that maximize the efficiency and safety of subsequent thermo-mechanical lysis procedures.

The device comprises a radiation source configured to emit pulsed electromagnetic radiation, an ultrasonic detector for detecting the optoacoustic waves generated by the interaction of the radiation with the skin, and a processing unit that analyses the detected signals to generate valuable data. It is noted that the optoacoustic signal may be processed to reveal the distribution of heat within various layers of the skin, such as the tattoo ink and melanin layers, as long as the wave receptor or transducer follows the same shape of said layers, such as a planar surface, and this information may thus be employed for predicting the outcome of a thermo-mechanical lysis treatment before application, and/or for optimize the laser parameters in said non-therapeutic treatment. This allows, for example, to avoid performing the non-therapeutic treatment if it is predicted a low efficiency in the tattoo or skin spot removal and/or it is predicted high probabilities of damaging the skin. The processing unit is in some embodiments programmed to calculate the deposited heat and, preferably, predict the outcome of the treatment, based on the distribution of energy between the tattoo ink and the melanin layer. The method and device further provide in some embodiments the tools for selecting optimal wavelengths, beam diameters, and laser pulse energies to guarantee efficient tattoo removal without causing damage to surrounding tissues.

The device and method of the invention are preferably designed to isolate the wave components of the signal belonging to the tattoo ink slab and/or from the melanin layer, thanks to the geometrical shape of the transducer, which follows the shape of the target layers, allowing it to receive the signal from said layers in phase and without interference. The device and method of the invention further provide in some embodiments a custom noise-removing signal filtering, which allows to obtain a clear representation of the optoacoustic wave generated in the ink layer and/or in the melanin-containing layer, and from this cleaned-out waveform the device and algorithm of the invention can readily extract characterizing parameters from the target layers, such as thickness, distance to the transducer and/or to the skin surface (once the distance to the skin surface is measured for example with the transducer), and, more importantly, the amount of deposited heat, preferably expressed as a percentage with respect to the deposited heat in a reference, wherein said reference may be an external layer of a high-absorptive material or a melanin layer in the skin. By using optoacoustic imaging, in some embodiments the system can predict whether excessive heating of the melanin layer could lead to adverse effects, such as skin whitening or burns, and adjust treatment parameters accordingly. The device is preferably configured to display an image or heatmap of the ink and/or melanin-containing layers, providing a clear visual representation of the heat distribution and allowing the practitioner to make informed decisions regarding the treatment.

In a first aspect of the invention, a device (1) for optoacoustic measurement of deposited heat in one or more target layers (21, 22) comprised in human or animal skin tissue is disclosed.

The device comprises a radiation source configured to emit pulsed electromagnetic radiation suitable for generating optoacoustic waves, an ultrasonic detector for detecting optoacoustic waves generated by the interaction of the emitted radiation with the one or more target layers, the ultrasonic detector comprising a transducer with a sensing surface, and a processing unit in communication with the radiation source and the ultrasonic detector, and in communication with a memory. The memory comprises instructions which, when executed by the processing unit, cause said processing unit to determine the distance between the sensing surface of the transducer and one or more of the target layers from the relationship between the time of the radiation emission, the optoacoustic wave received as a function of time, and the sound velocity. Optionally, if it is detected optoacoustic waves associated with more than one target layer, the processing unit is further configured to determine an indication of the relative distance between two or more of said target layers by analyzing the time difference of said optoacoustic waves. The processing unit is further configured to determine the thickness of one or more target layers by analyzing the detected optoacoustic waves associated with said target layers and to generate data and/or an image comprising information related to the deposited heat in the one or more target layers, their distance to the sensing surface of the transducer, and/or their respective thickness, wherein the one or more target layers comprise at least an ink layer and/or a melanin layer.

According to a preferred embodiment of the first aspect of the invention, the device is configured to be calibrated by measuring a reference optoacoustic wave from a reference element of a high light absorption coefficient material, preferably wherein the optoacoustic waves detected in step (b) are normalized or expressed in percentage compared with the reference wave, more preferably wherein the reference element is a reference layer.

According to a preferred embodiment of the first aspect of the invention, the memory comprises further instructions which, when executed by the processing unit, cause said processing unit to analyze the detected optoacoustic waves to determine the relative difference in deposited heat between any two of the ink layer, the melanin-containing layer, and the reference element, or between the three of them. The processing unit is further configured to generate data and/or an image comprising information related to the relative difference in deposited heat calculated in step (iii).

According to a preferred embodiment of the first aspect of the invention, the memory comprises further instructions which, when executed by the processing unit, cause said processing unit to generate a heatmap representing the amount of heat deposited in one or more target layers.

According to a preferred embodiment of the first aspect of the invention, the memory comprises further instructions which, when executed by the processing unit, cause said processing unit to select a wavelength that optimizes a ratio of deposited heat in the ink layer with respect to deposited heat in the melanin layer, by analyzing, for different wavelengths of the electromagnetic radiation pulses, the detected optoacoustic waves to extract the quantity of deposited heat for each wavelength at the ink layer and at the melanin-containing layer, and comparing the ratios of deposited heat by the melanin-containing layer and the ink layer.

According to a preferred embodiment of the first aspect of the invention, the device further comprises a removable piece, wherein the removable piece comprises the heat-absorbing material and/or the reference element, such that when the removable piece is attached to the device and the device is applied on the surface of the skin tissue for optoacoustic measurement, the heat-absorbing material and/or the reference element rests on the skin of said biological material, preferably wherein the heat-absorbing material is a black thread.

According to a preferred embodiment of the first aspect of the invention, the memory comprises further instructions which, when executed by the processing unit cause said processing unit to filter noise in the detected optoacustic waves by applying a noise filtering algorithm based on the following mathematical transform: ${C}_{{d}_{l}} = {\sum }_{n = - \left(M-1\right) / 2}^{\left(M-1\right) / 2} P \left({d}_{n}\right) {φ}_{{d}_{l}} \left({d}_{n}\right)$

Wherein *C_{dₗ}* are the coefficients of the components of the optoacoustic wave signal *P*(*dₙ*) expressed as a function of the distance to the detector *dₙ*, and the wave components of the signal are *φ_{dₗ}*(*dₙ*) wherein *φ_{dl}*(*dₙ*) can be expressed as: ${φ}_{{d}_{l}} \left({d}_{n}\right) = \left\{\begin{matrix}\frac{- 1}{\sqrt{2}} if {d}_{n} = {d}_{l} \\ \frac{1}{\sqrt{2}} if - {d}_{n} = {d}_{l} \\ 0 otherwise\end{matrix}\right.$

**Wherein** *dₗ* is the diameter of the wave emitter.

According to a preferred embodiment of the first aspect of the invention, the memory comprises further instructions which, when executed by the processing unit, cause said processing unit to calculate a probability estimate of the success of a thermo-mechanical lysis non-therapeutic procedure, based on the deposited heat in at least one of the target layers, wherein said probability is preferably calculated for at least two wavelengths of the radiation source and/or based on the comparison of the deposited heat in the ink layer and the deposited heat in the melanin layer and/or in the reference element.

According to a preferred embodiment of the first aspect of the invention, the memory comprises further instructions which, when executed by the processing unit, cause said processing unit to estimate granule size in the ink layer and/or in the melanin-containing layer from optoacoustic wave analysis, preferably before and after a thermo-mechanical lysis non-therapeutic procedure.

According to a second aspect of the invention, a non-therapeutic use of a device according to any one of the previous embodiments is disclosed, wherein said use is intended to predict the outcome of a thermo-mechanical lysis treatment and/or estimate skin damage or whitening associated with said thermo-mechanical lysis treatment, preferably wherein the thermo-mechanical lysis treatment is used for tattoo removal or for the removal of skin lentigines, melasma, or other dark spots related to aging.

According to a third aspect of the invention, a non-therapeutic method for determining the relative deposited heat between an ink layer comprised in skin tissue, a melanin-containing layer comprised in said skin tissue, and/or a reference element of a high light absorption material not comprised in said skin tissue is disclosed. The method comprises irradiating the skin tissue with pulsed electromagnetic radiation from a radiation source, detecting waves generated by the interaction of the radiation with the ink layer, the melanin-containing layer, and/or the reference element, wherein the detecting is made with an ultrasonic detector comprising a transducer with a sensing surface, wherein the sensing surface is planar or has the shape of the layer of ink or melanin and/or the shape of the surface of the skin tissue, and analyzing with a processing unit the detected optoacoustic waves to determine the relative difference in absorbed power between any two of the ink layer, the melanin-containing layer, and the reference element. Preferably, the method further comprises displaying on a display data and/or an image comprising information related to the relative difference in absorbed power, preferably wherein said image is a heat map representing the energy absorbed by any combination of the ink layer, the melanin-containing layer, and the reference element.

According to a preferred embodiment of the third aspect of the invention, the method further comprises obtaining optoacoustic waves at two or more wavelengths and determining the most suitable laser wavelength from the two or more wavelengths for a thermo-mechanical lysis treatment based on the ratio of heat deposited in any at least two layers of the melanin-containing layer, the ink layer, and the reference element, and/or based solely on the deposited heat in the ink layer.

According to a preferred embodiment of the third aspect of the invention, the method further comprises filtering noise in the detected optoacustic waves by applying a noise filtering algorithm based on the following mathematical transform: ${C}_{{d}_{l}} = {\sum }_{n = - \left(M-1\right) / 2}^{\left(M-1\right) / 2} P \left({d}_{n}\right) {φ}_{{d}_{l}} \left({d}_{n}\right)$

Wherein *C_{dₗ}* are the coefficients of the components of an the signal *P*(*dₙ*) expressed as a function of the distance to the detector *dₙ*, and the wave componennts of the signal are *φ_{dₗ}*(*dₙ*) wherein *φ_{dl}*(*dₙ*) can be expressed as: ${φ}_{{d}_{l}} \left({d}_{n}\right) = \left\{\begin{matrix}\frac{- 1}{\sqrt{2}} if {d}_{n} = {d}_{l} \\ \frac{1}{\sqrt{2}} if - {d}_{n} = {d}_{l} \\ 0 otherwise\end{matrix}\right.$

Wherein *dₗ* is the diameter of the wave emitter.

According to a preferred embodiment of the third aspect of the invention, the method further comprises calculating a probability estimate of the success of a non-therapeutic thermo-mechanical lysis procedure, based on the relative deposited heat in the ink layer and the melanin-containing layer. Preferably, the method further comprises displaying said probability estimate of the success of a laser treatment procedure and/or an image comprising visual information related to said probability estimate of the success of a laser treatment procedure.

According to a preferred embodiment of the third aspect of the invention, the method further comprises estimating granule size in the composition of the ink layer and/or in the melanin-containing layer before a thermo-mechanical lysis non-therapeutic procedure, estimating granule size in the composition of the ink layer and/or in the melanin-containing layer after a thermo-mechanical lysis non-therapeutic procedure, and comparing the granule size estimated before and after the laser non-therapeutic procedure to determine, based on said comparison, if the procedure was successful.

### Brief description of the drawings

To enable a better understanding of the present disclosure, and to show how the present disclosure may be carried out, reference will now be made, by way of example only, to the accompanying schematic drawings, wherein:

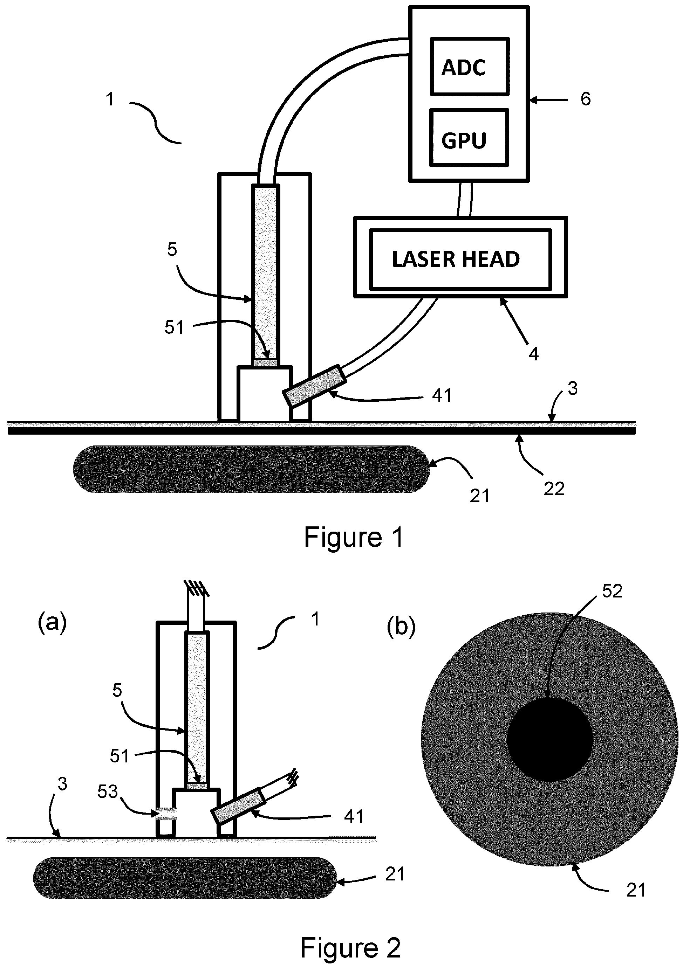

Figure 1 shows a schematic representation of a device for optoacoustic measurement of deposited heat in one or more target layers comprised in skin tissue according to one or more embodiments of the invention.
Figure 2 shows a schematic representation of (a) a device for optoacoustic measurement of deposited heat in one or more target layers comprised in skin tissue, wherein the device further comprises an aperture to allow excess gel placed in between the skin of the biological tissue and the transducer to exit, preventing thus the formation of bubbles, and (b), a bottom view of a target layer and the surface of said device according to one or more embodiments of the invention.

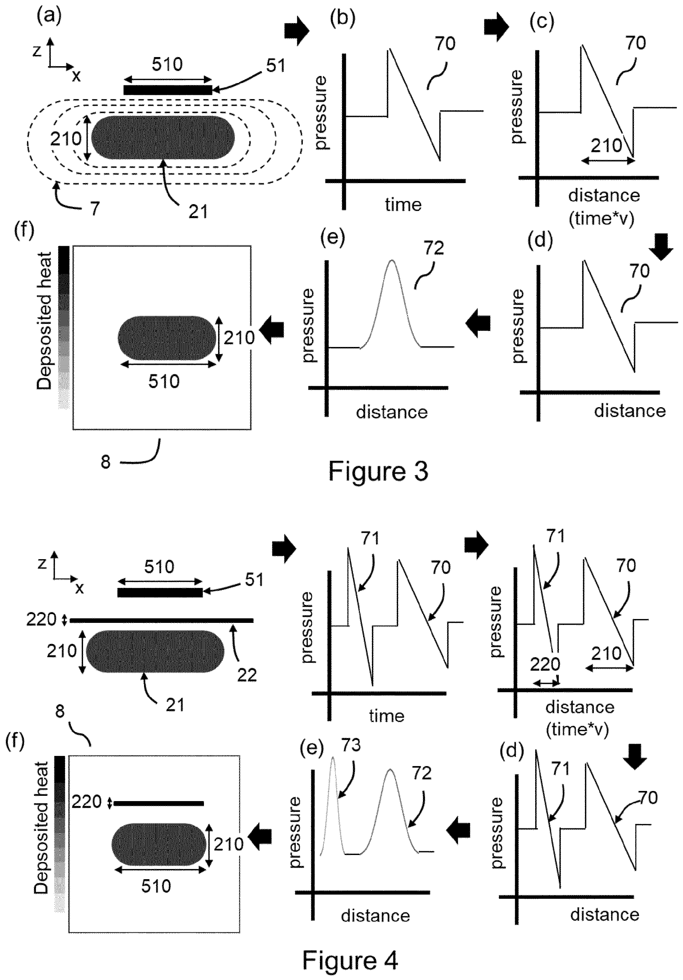

Figure 3 shows a schematic representation of the generation of optoacoustic waves in a medium, such a skin tissue, by heat deposition in an ink layer (3a) comprised in said medium, the pressure profile induce by said optoacoustic wave as a function of time (3b) and as a function of distance (3c and 3d), the envelope of said wave (3e), and a visual representation of the thickness and heat deposited in the slab or layer of ink that generated the optoacoustic wave (3f), wherein the width is the width of the measuring transducer, according to one or more embodiments of the invention.
Figure 4 shows a schematic representation of the generation of optoacoustic waves in a medium by heat deposition in an ink layer and in a melanin-containing layer (4a) comprised in said medium, the pressure profile induce by said optoacoustic waves as a function of time (4b) and as a function of distance to the transducer (4c and 4d), the envelope of said waves (4e), and a visual representation of the thickness and heat deposited in the slab or layer of ink and in the layer of melanin (4f), that generated the optoacoustic wave, wherein the width shown is the width of the measuring transducer, according to one or more embodiments of the invention.

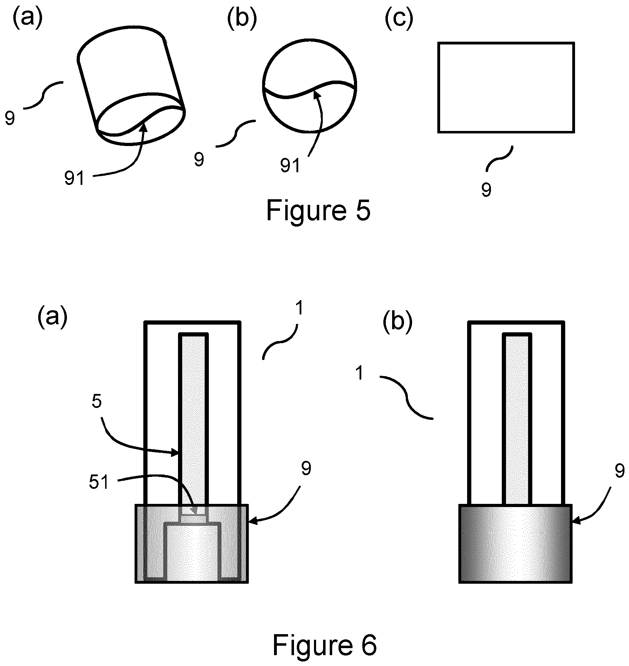

Figure 5 shows a removable piece comprising a heat absorbing material in the shape of a thread, in (a) perspective view, (b) Bottom view, and (c) lateral view, according to one or more embodiments of the invention.
Figure 6 shows part of a device for optoacoustic measurement according to one or more embodiments of the invention comprising a removable piece which comprises the heat absorbing material in the shape of a thread, in (a) a semi transparent view of the removable piece, and (b) opaque view of said removable piece.

### Description of the invention

### Definitions

As used herein, "a" or "an" means "at least one" or "one or more."

It is noted that the term "about", if used herein, refers to +/- 30%, preferably +/- 20%, preferably +/- 15%, more preferably +/- 10%, of the indicated referred value. It may also refer to the exact value, that is, +/- 0% or +/- the standard measurement error. Also, any quantity, composition percentages and/or dimension mentioned herein is understood to present an error of +/- 30%, preferably +/- 20%, preferably +/- 15%, more preferably +/- 10%, more preferably +/- 5% or +/-0% of the indicated value. It may also refer to the exact value, that is, +/- 0% or +/- the standard measurement error.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together.

When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

Unless defined otherwise, all technical and scientific terms used herein have the same meaning as is commonly understood by one of ordinary skill in the art to which this invention belongs. All patents, applications, published applications and other publications referred to herein are incorporated by reference in their entirety. If a definition set forth in this section is contrary to or otherwise inconsistent with a definition set forth in applications, published applications and other publications that are herein incorporated by reference, the definition set forth in this section prevails over the definition that is incorporated herein by reference.

The term "optoacoustic wave", in the context of the invention, refers to a type of wave generated through the interaction of light (usually in the form of pulsed laser or other optical sources) with a target material, such as biological tissue or, preferably, a target layer comprised in said tissue. This interaction results in the absorption of light, followed by rapid thermal expansion of the material, generating an acoustic wave. This acoustic wave can be measured to provide information about the tissue's optical properties, thermal response, and mechanical characteristics. In some embodiments, the optoacoustic wave may be used as a non-therapeutic signal to monitor and assess the heat-induced effects on the skin or tissue, for procedures such as removal of tattoos or skin spots, or to predict its efficacy. The frequency, intensity, and propagation characteristics of the optoacoustic wave are linked to the composition and structure of the underlying tissue or layer, such as its thickness, distance to the optoacoustic receptor, and heat deposited at said layer for a given wavelength, allowing thus in some embodiments to characterize and parametrize any pigment layers such as ink-containing layers and/or melanin-containing layers.

The term "optoacoustic measurement", in the context of the invention, refers to the process of detecting and analysing acoustic waves generated by the interaction of light with biological tissue. This measurement involves the use of optoacoustic sensors or transducers that convert the generated acoustic wave into an electrical signal, which can then be processed to assess tissue properties such as optical absorption or deposited heat, depth and thickness, among others characteristics. Specifically, in the context of the invention, optoacoustic measurement is used to monitor and control the deposition of heat within the skin, such as during thermo-mechanical lysis for tattoo or skin spot removal. In this context, the invention provides a transducer surface that follows the shape of the ink slab of the tattoo or the melanin-containin layer, in particular embodiments such as a planar surface are provided, for example a circular planar surface. It is noted that bigger optoacoustic sensors may employ transducer surfaces that bend to follow the shape of a tattoo or skin spot that is not planar, but for example convex such as those located in highly curved parts of the body. The measurement may involve the analysis of the temporal and spatial distribution of the optoacoustic signal, and according to some embodiments, can be used to provide insight into the effectiveness and safety of the procedure.

The term "human or animal skin tissue", in the context of the invention, refers to the biological tissue forming the outer covering of the body in humans and animals. This includes all structural layers of the skin, specifically the epidermis, dermis, and any underlying layers that directly interact with or support the skin, such as the hypodermis (subcutaneous tissue). It is noted that said dermal and epidermal layers of the skin may contain various substances such as ink, and/or cellular components such as melanin, which may be arranged in the form of "slabs", i.e., presenting a planar distribution or following the shape of the skin or below the skin surface. In particular, the skin tissue as referred to in the invention comprises one or more layers beneath the surface, such as those containing pigment deposits (e.g., from tattoos or skin spots), wherein said layers may be understood as high-density distributions of said pigment deposits or particles. For example, the tissue could include any skin tissue that contains either artificial or natural pigmentation, such as in the case of tattoos, hyperpigmented spots, or other forms of localized or unlocalized pigmentation, such as normal melanin distributions.

The term "surface of the skin tissue", in the context of the invention, refers to the outermost exposed layer of human or animal skin that serves as the boundary between the organism and its environment. This surface is typically composed of the epidermis, the outermost layer of skin. It is understood, in the context of the invention, that beneath this exposed surface, there may be underlying layers of skin that contain various types of pigmentation or other components. For example, beneath the skin surface, there may be a layer containing tattoo ink or an area of skin with a higher concentration of melanin, which could contribute to a visual pigmentation or spot.

The term "target layers", in the context of the invention, refers to the specific regions or depths within biological tissue that are targeted for treatment or analysis, preferably containing light-absorbing pigments, particularly in the context of optical and thermal interactions. These layers may lie beneath the skin surface and can include, for example, regions containing tattoo ink or areas with varying levels of pigmentation such as melanin. The target layers are the layers where selective thermo-mechanical lysis is intended to occur, or where the optoacoustic measurements are focused to assess the response of the tissue to light and heat application. The specific target layers may vary depending on the desired treatment outcome, for example, focusing on tattoo ink layers for removal or on deeper layers for skin spot treatment. These layers may extend across multiple tissue depths within the dermis and epidermis, and are selected based on their optical absorption properties and their relevance to the therapeutic or diagnostic procedure being employed. It is noted that preferably the term "target layer" refers in some embodiments to one or more layers, slabs or planar distributions of pigments comprised within the skin, typically within the dermal layer, such as, but not limited to, ink from a tattoo, melanin from a high melanin concentration skin spot, and/or melanin from a homogeneous concentration of melanin in the skin. A pigment layer may vary in thickness, depth, and distribution depending on the melanin concentration in the skin or in part of said skin, or the tattooing technique and the composition of the ink. In the context of the invention, the pigment layer represents a region that may be the targeted in a non-therapeutic treatment, such as in selective thermo-mechanical lysis, where the melanin deposits, or ink granules are subjected to heat or laser energy in order to break it down or remove it, as smaller granules can be assimilated by the immune system. Alternatively, said pigment layer may not be the target of the non-therapeutic treatment, and thus its energy absorbance or the quantity of heat deposited in said layer may be studied to determine if a treatment is going to work as intended or is going to be harmful because the energy will be absorbed by the non-target layer. The pigment layer may also exhibit different physical and optical properties compared to the surrounding skin tissue, which allows for its specific targeting during treatment.

The term "ink layer", in the context of the invention, refers to a layer of tattoo ink deposited within the skin, typically within the dermal layer, where the pigment resides following the tattooing process. This ink layer may comprise various pigments and substances used in the tattoo ink, which are introduced into the skin through needle penetration. The ink layer may vary in thickness, depth, and distribution depending on the tattooing technique and the composition of the ink. In some embodiments of the invention, the ink layer may represent the region that is targeted for treatment, such as in selective thermo-mechanical lysis, where the tattoo ink is subjected to heat or laser energy in order to break it down or remove it. The ink layer may also exhibit different physical and optical properties compared to the surrounding skin tissue, which allows for its specific targeting during treatment

The term "melanin-containing", in the context of the invention, refers to biological tissue that contains melanin, a natural pigment found in human and animal skin, as well as hair and eyes. Melanin is responsible for the coloration of the skin and plays a protective role against UV radiation. In the context of the invention, melanin-containing tissue may also refer to areas of skin or biological tissue that exhibit higher concentrations of melanin, such as in cases of hyperpigmentation, freckles, or other skin spots. Some embodiments of the invention may specifically target these melanin-rich regions using light and heat in an aesthetic process of selective thermo-mechanical lysis, which can be employed for non-therapeutic treatment purposes, such as the removal or alteration of skin spots or pigmentation. Melanin-containing areas may be located within the epidermal and dermal layers of the skin and are typically characterized by their optical absorption properties, making them responsive to light-based therapies.

The term "transducer with a sensing surface", in the context of the invention, refers to a device that is capable of converting mechanical or acoustic waves into an electrical signal, or vice versa, and has a surface that is directly involved in the detection of the signal. The sensing surface of the transducer is the portion of the device that comes into direct contact with the biological tissue or with a material with a similar acoustic impedance placed in between the biological tissue and the sensing surface, such as a gel, to minimize signal reflection and ensure efficient transmission of acoustic waves. Said sensing surface is thus is positioned in close proximity to the biological tissue, enabling the transducer to receive acoustic waves, and also further enabling it to emit and receive ultrasound signals to detect distances of target layers and/or the distance of the skin to the sensing surface, using the portion of the acoustic wave that would be reflected in the skin surface and the time to reach back to the position of the emitting sensing surface. This surface is preferably sensitive to the specific acoustic or optoacoustic signals generated by the tissue, such as those arising from the interaction of light with the skin or underlying layers. The sensing surface is typically designed to facilitate optimal coupling with a target layer, and by comprising a planar surface, or a surface shape that mimics the shape of the skin to be measured, it allows the optoacoustic waves generated by the heat deposition in the target layers to be received in phase and without destructive interferences, preferably if the sensing surface is placed over a central part of the target layer and not over the edges. In the context of the invention, the transducer with a sensing surface is preferably used to detect optoacoustic waves generated during treatments such as, but not limited to, tattoo removal, skin whitening, or skin spot reduction, and to provide real-time feedback for treatment monitoring.

The term "processing unit", in the context of the invention, refers to a component or system within the device that is responsible for processing, analysing, and/or controlling data and signals. The processing unit may include or be in communication with a controlling unit, wherein the controlling unit may manage operations such as the activation or synchronization of a radiation source and ultrasonic detector. In some embodiments, a processing unit may be involved in tasks like signal analysis and computation, whereas a controlling unit may be involved on device control and coordination, or, in alternative embodiments, all these tasks may be carried out by the processing unit. The processing unit may comprise a variety of components, including but not limited to, central processing units (CPUs), graphical processing units (GPUs), circuit boards, analog-to-digital converters (ADCs), and/or other processing hardware capable of handling data input and output. In some embodiments, the processing and controlling functions may be integrated into a single unit or may be modular, with communication protocols linking them together.

The term "deposited heat", in the context of the invention, refers to the electromagnetic energy that is absorbed and retained as heat within the target layers of the biological tissue during the application of an external energy source, such as light or ultrasonic radiation. This heat is absorbed by the tissue and may cause local temperature increases within specific regions, depending on factors such as the material properties of the tissue and the energy input. The deposited heat may be used as an indicator of the extent of thermal effects, such as fragmentation of pigment particles related to removal of ink or melanin-containing layers, tissue burning, and/or potential changes to the structure. In some embodiments the measurement of this heat allows to characterize one or more target layers, helping monitoring and anticipating the effects of a selective thermo-mechanical lysis.

The term "reference layer of a high light absorption coefficient material", in the context of the invention, refers to a material, which may be placed adjacent to or be comprised in the device, that has a high capacity for absorbing light at specific wavelengths. This reference layer can be used in some embodiments to calibrate the readings obtained from the target layers by providing a baseline or 100% absorption value for comparison. For example, a carbon sheet or similar material with high light absorption properties may be employed to simulate a high absorption scenario, which allows for accurate interpretation of measurements taken from the target layers.

The term "heatmap", in the context of the invention, refers to a visual representation of the distribution of thermal energy or heat deposition within the target layers of biological tissue, derived from optoacoustic measurements. The heatmap can display in some embodiments information about the position, thickness, and relative energy absorption or heat deposition of the target layers, with, for example, a two colour scale representing varying levels of heat absorption. For instance, regions with higher absorption may be displayed in warmer colours, such as red, or black, while areas with lower absorption may be represented in cooler colours, such as blue, or white. The heatmap provides a graphical representation of how the energy is distributed across the biological tissue and can also reflect the depth and thickness of the target layers, and it may be shown in 1D, wherein the axis is the depth or distance to the sensing surface of the transducer, in 2D, if taken in consideration also the width of the sensing surface, or in 3D, if taking bot dimensions of the surface of the sensing surface. It is preferably used to guide non-therapeutic treatment decisions by showing where the heat has been deposited relative to the underlying structures of the biological tissue.

The term "thermo-mechanical lysis", in the context of the invention, refers to a treatment process that utilizes targeted electromagnetic radiation to deposit heat, breaking down or fragmenting certain target components within biological tissues, particularly in the context of tattoo removal or the treatment of hyperpigmented spots. In tattoo removal, the heat is intended to be preferentially absorbed by the ink layer, leading to the breakdown of the ink particles into smaller fragments, which can then be naturally cleared by the body's immune system. In this sense, it is important that the heat is precisely delivered to the ink layer, with minimal heat deposition into the surrounding tissue, to prevent damage to the skin and to avoid undesirable side effects, such as burns or scarring. Similarly, in the context of melanin-containing skin spots (e.g., hyperpigmentation), the melanin-rich regions are preferentially targeted without causing harm to the surrounding skin tissue. Excessive heat absorption in the melanin-containing layer could result in unwanted whitening or even burns.

### Description

Current laser-based thermo-mechanical lysis technologies for non-therapeutic, aesthetic treatments have limitations. Laser settings are often determined through trial and error, relying on visual assessments of the skin's reaction, which provides limited feedback. Aditionally, the effectiveness of treatments is only clear weeks later, making it hard to gauge immediate results. Wavelength selection remains largely empirical, and choosing the right wavelength is challenging, especially with darker skin tones, where melanin can interfere with laser absorption. There are also significant safety concerns, particularly the risk of skin damage, like burns or scarring, especially for individuals with higher melanin concentrations or deeply embedded tattoos. This is a problem also for coloured tattoos, which require wavelengths that are absorbed well by melanin or other natural chromophores.

Laser spot size optimization and real-time feedback for laser parameters are other unresolved issues. These challenges hinder precision in adjusting energy levels and assessing treatment effectiveness. Currently, there's no reliable system to measure and predict heat absorption, ink fragmentation, or tissue response during the procedure, which could lead to adverse effects. More importantly, there are no current solutions that provide an estimation of how much heat is going to be absorbed by one or more target pigment layer, to determine a probability of success of the treatment, or to compare between, for example, an ink layer and a melanin-containing layer, to determine if the tattoo is going to absorb most of the power, facilitating the fragmentation of its pigments and promoting its disappearance by the interaction of these smaller particles with the immune system, or if, on the other hand, most of the energy is going to be deposited as heat in the melanin layer or the rest of the biological tissue, prompting and unwanted whitening or a burn or scarification.

In this invention we disclose a device that addresses the aforementioned issues and successfully solves them.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

In a first aspect of the invention, a device (1) for optoacoustic measurement of deposited heat in one or more target layers (21, 22) comprised in human or animal skin tissue is disclosed. For example, said one or more target layers (21, 22) may be comprised in the dermis, below the epidermis and below the surface of the skin (3), wherein the surface of the skin (3) is understood to define the boundary of the skin tissue with the external world. It is noted that the device (1) of the invention comprises:
(a) a radiation source (4) configured to emit electromagnetic radiation suitable for generating optoacoustic waves (70, 71), preferably wherein the radiation source (4) can emit pulsed electromagnetic radiation;
(b) an ultrasonic detector (5) for detecting optoacoustic waves (70, 71) generated by the interaction of the emitted radiation with the one or more target layers (21, 22) (2), the ultrasonic detector (5) comprising a transducer (51) with a sensing surface; and
(c) a processing unit (6) in communication with the radiation source (4) and the ultrasonic detector (5), and in communication with a memory.

Furthermore, the memory, which preferably is comprised in the device (1) of the invention, comprises instructions which, when executed by the processing unit (6) cause said processing unit (6) to:
(i) determine the distance between the sensing surface of the transducer (51) and one or more of the target layers (21, 22) from the relationship between the time of the radiation emission, the optoacoustic wave received as a function of time and the sound velocity;
(ii) preferably, if it is detected optoacoustic waves (70, 71) associated to more than one target layer (21, 22), determine an indication of the relative distance between two or more of said target layers (21, 22) by analysing the time difference of said optoacoustic waves (70, 71);
(iii) determine the thickness of one or more target layers (21, 22), by analysing the detected optoacoustic waves (70, 71) associated to target layers (21, 22)said target layers (21, 22); and
(iv) generate data and/or an image comprising information related to the deposited heat in the one or more target layers (21, 22), their distance to the sensing surface of the transducer (51) and/or their respective thickness.

It is further noted that the one or more target layers (21, 22) comprise at least an ink layer (21), such as from a tattoo, and/or a melanin layer (22), such as from a high pigmentation spot.

The radiation source (4) may comprise, but is not limited to, lasers such as Nd:YAG lasers, diode lasers, alexandrite lasers, ruby lasers, picosecond lasers, femtosecond lasers, or other pulsed or continuous electromagnetic radiation sources that emit radiation within wavelengths optimized to interact selectively with pigments such as tattoo inks or melanin. In some embodiments the radiation pulses may be adjustable in energy (e.g., in a range from about 1 mJ to several Joules), pulse duration (typically ranging from femtoseconds to microseconds), and wavelength (ranging for in some embodiments between 400 nm and 1200 nm), allowing a tailored approach for different types of pigments and skin conditions. Preferably, the laser is not tuneable, wherein the wavelength is fixed, making it more manageable and durable, but in some embodiments the laser is tuneable. Additionally, the spot size of the laser may be adaptable in some embodiments by employing optical elements, such as lenses, prisms, collimators, and/or diaphragms, among other options. Preferably, the radiation source emits pulsed electromagnetic radiation.

It is further noted that a pulsed radiation source (4) is preferred not only because delivering a high energy density within a very short time interval induces rapid thermoelastic expansion and thus generates clearly detectable optoacoustic waves, but also because the short pulse duration advantageously minimizes heat diffusion into surrounding tissues. Specifically, when pulses have sufficiently short durations-typically from femtoseconds to nanoseconds-the deposited heat remains substantially confined within the targeted pigment granules or layers, a phenomenon known as thermal or heat confinement. This confinement prevents undesirable heat propagation beyond the targeted region, significantly reducing the risk of thermal damage, such as burns or unwanted tissue destruction, in adjacent non-targeted structures. Consequently, the selective interaction with targeted pigments, such as tattoo inks or melanin, ensures safer and more effective outcomes for thermo-mechanical lysis procedures.

It is further noted that the sensing surface of the transducer (51) in some embodiments may have a geometric shape designed to match or approximate the geometry of the target layers (21, 22), which are typically planar, following the shape of the skin, but in some cases may be curved, such as at the elbow, shoulder, and any other highly curved part of the body. Following the shape of the target layers (21, 22) allows the sensing surface of the transducer (51) to enhance the detection of optoacoustic waves by promoting its collection in phase, wherein no border effects, diffraction effects or interferences, among other possible optical phenomena, happen due to dissimilarities in shape of the emitter and the receiver. Preferably the wavefront received is, thus, planar and/or in phase due to matching the emitter and receiver surface. Alternatively, the transducer (51) may comprise multiple sensing elements arranged in an array configuration, for instance linear, circular, annular, or matrix-type arrays, preferably circular enabling improved spatial resolution, depth discrimination, or multidimensional imaging capability. In some embodiments, comprising multiple sensing elements allows the transducer (51) to adapt to the shape of the skin tissue in different parts of the body of the human or animal. Suitable transducer materials may comprise, but are not limited to, piezoelectric ceramics such as lead zirconate titanate (PZT), piezoelectric polymers like polyvinylidene fluoride (PVDF), capacitive micromachined ultrasonic transducers (CMUTs), or piezocomposite materials, selected according to desired sensitivity, frequency response (e.g., ranging from a few MHz to tens of MHz), and spatial resolution.

In some embodiments, the processing unit (6) of the device (1) is configured to communicate directly with the radiation source (4) and the ultrasonic detector (5) and/or to control them. Alternatively, the device (1) may further comprise a controlling unit, which may be integrated with or separate from the processing unit (6). Such a controlling unit may manage the synchronization between the emission of radiation pulses and the subsequent detection of optoacoustic signals, ensuring precise temporal coordination to accurately correlate optoacoustic data with emitted pulses. This controlling unit, when separate, is preferably in communication with the processing unit (6), the radiation source (4), and the ultrasonic detector (5) via wired connections (such as electrical cables, fibre optics, or coaxial connections, among other options) or wireless connections (for instance Wi-Fi, Bluetooth, radio frequency communication, or infrared communication).

The processing unit (6), preferably comprising at least one microprocessor, microcontroller, FPGA (field-programmable gate array), ADC (analogue-to-digital converter), CPU (central processing unit), GPU (graphics processing unit) or DSP (digital signal processor), is configured to execute processor-readable algorithms stored within the memory to analyse the detected optoacoustic signals (70, 71). The memory may comprise RAM, ROM, flash memory, solid-state drives, or magnetic storage, and store instructions for performing analytical tasks such as calculating the distance from the transducer sensing surface to the one or more target layers (21, 22). This calculation preferably employs the known or estimated speed of sound propagation within skin tissues (typically ranging from approximately 1450 m/s to 1650 m/s), and correlates the temporal delay between radiation pulse emission and the arrival of optoacoustic signals at the transducer (51). It is not that the speed of light as a parameter is generally no necessary in such calculations, as it is several orders of magnitude faster than the speed of sound, however, in some embodiments it could be accounted for. Said memory is preferably comprised in the device (1) of the invention, but it could be stored somewhere else, such as in the cloud, and be in communication with the processing unit (6) via wire or wireless.

In some embodiments, the device (1) may be configured to determine relative distances between multiple target layers (21, 22), allowing in some embodiments to distinguish the ink layer (21) from the melanin layer (22), by analysing the difference in arrival times of their respective optoacoustic waves (70, 71) and/or their shape or features. Additionally, the thickness of these layers may be computed by analysing the shape, duration, and spectral content of the optoacoustic waves (70, 71), taking advantage of characteristic acoustic signatures associated with varying optical absorption and thermal expansion properties of the different layers, such as the distance between a local or global maximum and a local or global minimum pressure point in time and/or in spatial coordinate.

It is noted that the device (1) may provide generated data in various forms, including numeric parameters, textual descriptions, graphical representations, or combinations thereof. Preferably, the processing unit (6) is configured to output these data to a graphical user interface (GUI), accessible to an operator or medical practitioner via a monitor, touchscreen, handheld device, tablet, computer, smartphone, or augmented reality display, which in some mebodiments is comprised in the device (1) of the invention. Alternatively or additionally, generated data may be provided audibly, or in electronic formats suitable for integration with external systems such as medical record databases, treatment planning software, or cloud-based platforms for remote analysis.

The generated data comprise, preferably, images or heatmaps displaying a spatial distribution of deposited heat within the targeted layers (21, 22), illustrating in some embodiments the intensity and localization of heating as quantified by the optoacoustic analysis. The images representing distances and thicknesses may be presented in one-dimensional, such as a line departing from the skin surface until reaching one or more target layers, two-dimensional, for example by taking in consideration the width of the sensing surface of the transducer (51) or three-dimensional formats, for example if considering both axis of the planar surface of the transducer (51). The images may employ color-coded maps, grayscale images, or overlay visualizations that clearly distinguish between layers such as ink deposits or melanin concentration. Additionally, textual or numeric data may further provide quantitative information such as percentages of heat deposition relative to a reference, absolute energy deposition values, calculated thicknesses, or distances from the skin surface.

In further embodiments, the device (1) may be configured for employing advanced signal processing techniques such as Fourier analysis, wavelet transforms, artificial intelligence algorithms including neural networks, or statistical classifiers to isolate and characterize relevant optoacoustic wave components. Other noise-reduction filters, digital signal filters, band-pass filtering, matched filtering, or adaptive filtering methods may also be employed to enhance the clarity and accuracy of optoacoustic measurements, thereby improving the reliability and precision of the predictive data and images generated.

It is further noted that, in some embodiments of the invention, the radiation source (4) is a pulsed radiation source (4) that preferably has a pulse duration substantially identical to the pulse duration of the laser employed in a subsequent selective thermo-mechanical lysis device used for a non-therapeutic treatment, such as tattoo removal or skin whitening. In some embodiments, the pulse length of the radiation emitted by the radiation source (4) preferably lies within the range of 10 picoseconds (ps) to 100 nanoseconds (ns). Preferred embodiments may thus comprise pulse durations such as, but not limited to, 10 ps, 100 ps, 500 ps, 1 ns, 10 ns, 50 ns, or 100 ns, depending on the nature of the targeted skin layers (21, 22). Moreover, the pulse energy emitted by the radiation source (4) per pulse may range between about 10 picojoules (pJ) and 100 microjoules (µJ), for instance, 50 pJ, 100 pJ, 1 nJ, 10 nJ, 100 nJ, 1 µJ, 10 µJ, or 50 µJ, among other suitable intermediate values. Such relatively low-energy pulses allow minimal heat deposition within the targeted skin structures, while still generating a detectable optoacoustic wave (70, 71) through thermoelastic expansion, thereby providing accurate, sensitive, and informative measurements without causing significant thermal damage.

It is additionally noted that the optoacoustic wave (70, 71) produced by the radiation source (4) through thermoelastic expansion may carry quantitative and spatial information regarding the deposited heat in the absorbing structures, such as a tattoo ink layer (21) and/or a melanin layer (22). In preferred embodiments, the processing unit (6) and/or the controlling unit, may use the information contained within the optoacoustic waves (70, 71) to determine optimal parameters for subsequent high-energy lasers employed by selective thermo-mechanical lysis devices. These parameters may comprise, but are not limited to, optimal wavelength selection, energy per pulse, beam diameter, pulse duration, and repetition rate, thereby enabling precise predictions of the efficiency and safety of the anticipated selective thermo-mechanical lysis treatment.

It is further noted that, due to the advantageous planar shape or selected geometry or shape of the sensing surface of the transducer (51), preferably following that of one or more target layers, the device (1) may significantly enhance detection selectivity and sensitivity, particularly toward the optoacoustic signals (70, 71) originating from the tattoo ink layer (21). The planar or geometrically adapted shape of the transducer (51) surface facilitates stronger detection of optoacoustic signals (70) from the ink layer (21), compared to signals originating from surrounding tissues such as microvessels or non-targeted skin structures. Accordingly, in some embodiments, the processing unit (6) may be configured to discriminate and isolate the optoacoustic signals (70) corresponding specifically to the tattoo ink layer (21) by employing a thresholding method. In one advantageous embodiment, this discrimination may be performed by applying a signal threshold set to a predefined percentage, for instance at or above approximately 25% of the maximum detected optoacoustic signal amplitude. Alternative embodiments may set this threshold at values within the range from about 0% to about 70%, such as 10%, 20%, 30%, 50%, or 60%. Alternatively or additionally, the threshold value may be dynamically determined using a reference optoacoustic signal obtained from an efficient, highly absorbing reference material which may be integrated into the device (1) or external, for example placed over the skin, thus further enhancing discrimination accuracy and consistency across varying skin types and conditions.

In some embodiments, the processing unit (6) of the device (1) is advantageously configured to generate a detailed spatial image or representation of the tattoo ink distribution (21) based on the detected optoacoustic signals or waves (70, 71). For example, when the transducer (51) comprises a disc-shaped sensing surface, the resulting spatial image may correspond to a three-dimensional cylindrical slab of the tattoo ink layer (21). The diameter of said cylindrical slab would correspond substantially to the diameter of the disc-shaped transducer (51), while the depth profile (z-direction) may be derived from the intensity and temporal characteristics of the detected optoacoustic waves (70, 71). The depth information can be computed by multiplying discrete time intervals of the optoacoustic signals or waves (70, 71) by the acoustic propagation velocity within the skin tissue, typically between 1450 m/s and 1650 m/s. To enhance visualization clarity, the envelope of the detected optoacoustic signals (70) may be calculated using a Hilbert transform or alternative envelope-detection methods, thereby creating an accurate and intuitive representation of the three-dimensional ink distribution within the target skin layers.

Additionally, it is noted that, in some embodiments of the first aspect of the invention, the processing unit (6) may calculate the deposited heat within the tattoo ink layer (21) by analysing various characteristics of the detected optoacoustic waves (70, 71). Such calculations may advantageously include, but are not limited to, peak-to-peak signal amplitude, signal energy, intensity distribution, area under the curve, integral of the signal envelope, or other suitable measures derived from the optoacoustic waveform. These calculated values may also be directly represented visually in the previously described spatial image or heatmap generated by the processing unit (6), facilitating intuitive interpretation of heat distribution and improving the predictive accuracy of the subsequent selective thermo-mechanical lysis treatments.

Advantageously, the device (1) of the invention provides a comprehensive, accurate, and non-invasive approach to predict and optimize outcomes of selective thermo-mechanical lysis treatments, specifically in non-therapeutic applications such as tattoo removal, removal of skin spots with high melanin concentrations, or skin whitening. By enabling precise quantification of heat deposition within target skin layers prior to actual treatment, the invention provides an invaluable to assess the adequacy of the treatment before performing it, while significantly improving safety and efficacy, allowing to reduce the likelihood of adverse effects like burns or unwanted pigmentation changes. Furthermore, the device (1) and method of the invention allows customization of thermo-mechanical lysis treatment parameters, and provides real-time feedback to practitioners. The invention, thus, provides an invaluable tool to determine if enough heat is going to be deposited in a target layer, and if a quantity of heat below a safety threshold is going to be deposited in other target layers, allowing thus to determine if the target layer intended to be removed is going to absorb enough power, while other layers that need to remain undamaged are going to absorb power below a safety threshold, thus allowing the user to prevent whitening and burn, among other unwanted side effects.

According to a preferred embodiment of the first aspect of the invention, the device (1) is configured to be calibrated by measuring a reference optoacoustic wave from a reference element of a high light absorption coefficient material, preferably not naturally comprised in the skin tissue, preferably wherein the optoacoustic waves (70, 71) detected in step (b) are normalized or expressed in percentage compared with the reference wave, more preferably wherein the reference element is a reference layer.

In some embodiments, the reference element used for calibration may comprise materials characterized by particularly high optical absorption coefficients. Such materials may comprise, but are not limited to, carbon-based materials such as carbon sheets, carbon black layers, graphite films, or carbon threads. Other suitable alternatives may comprise materials with black foils or black coatings, or dyed polymeric materials exhibiting high optical absorption within the relevant wavelengths emitted by the radiation source (4). The reference element may also comprise metals, semiconductor materials, metallic oxides, or composites designed specifically for stable and repeatable optoacoustic signal generation.

In some embodiments of the invention, the reference element may be arranged in different configurations with respect to the device (1). For instance, it may be integrally comprised within the device (1), permanently embedded into its housing or into a dedicated calibration module, or in a movable piece. Alternatively, the reference element may be provided within a removable portion or attachment of the device (1), allowing convenient insertion or removal during calibration phases. In other advantageous configurations, the reference element could be implemented as a separate independent piece, which may be positioned manually or automatically at an optimal distance from the transducer (51) such as resting on top of the skin to be measured, prior to calibration procedures.

It is further noted that, during calibration, the optoacoustic waves (70, 71) measured from the skin tissue may be normalized or expressed as a percentage relative to the amplitude or energy of the reference optoacoustic wave. Allowing thus reliable compensation of variations in device performance due to ambient conditions, aging of the device components, or minor misalignments between the radiation source (4) and transducer (51). This normalization may be carried out automatically by the processing unit (6) or, alternatively, through external software or dedicated calibration routines implemented periodically or before each measurement session.

Advantageously, the calibration of the device (1) using a high-absorption reference element ensures consistently accurate optoacoustic measurements, enhancing the reliability and reproducibility of the quantitative analyses of heat deposition within the targeted layers (21, 22). This precise calibration can significantly reduce measurement errors, facilitating standardization across multiple devices, and contributing to more predictable and optimized outcomes of the subsequent selective photothermolysis treatments.

According to a preferred embodiment of the first aspect of the invention, the memory comprises further instructions which, when executed by the processing unit (6), cause said processing unit (6) to:
v. analyse the detected optoacoustic waves (70, 71) to determine the relative difference in deposited heat between any two of the ink layer (21), the melanin-containing layer, and the reference element, or between the three of them; and to
vi. generate data and/or an image comprising information related to the relative difference in deposited heat calculated in step (iii).

It is noted that, in some embodiments of the invention, the processing unit (6) may further be configured to evaluate and determine the relative heat deposition differences when multiple ink layers (21) or multiple melanin-containing layers (22) are present within the targeted skin area. Such embodiments may encompass various combinations of layers, such as multiple tattoo inks of distinct colours, pigments, densities, or depths, or multiple melanin layers with varying pigment concentration or thickness. In such cases, the processing unit (6) may independently analyse each detected optoacoustic wave (70, 71), and compute differences in deposited heat among these multiple layers, allowing detailed characterization of complex skin conditions.

It is further noted that the generated data and/or image depicting the relative differences in deposited heat may be expressed in multiple forms, such as numerical ratios, percentages, differential heat maps, graphical plots, or comparative tables. Preferably, these representations are displayed visually on a graphical user interface, enabling practitioners to rapidly and intuitively assess the distribution of heat deposition across different layers. Alternative methods of data representation include text-based reports summarizing the relative heat deposition or exporting data in formats compatible with external analysis and reporting systems.

In some embodiments, the analysis performed by the processing unit (6) to calculate the relative differences in deposited heat may utilize advanced computational algorithms, such as, but not limited to, mathematical normalization, subtraction of waveforms, ratio-based comparisons, or differential signal processing techniques. The calculations may be based on direct analysis of raw optoacoustic waveforms or derived envelopes, spectral components, peak amplitudes, or signal integrals, providing flexibility in interpreting the data to accommodate varying clinical or experimental requirements.

Advantageously, by generating detailed comparative heat deposition information among the ink layer (21), melanin-containing layer (22), and reference element, the device (1) enables precise and informed prediction of treatment outcomes. This capability significantly improves procedural safety by identifying scenarios where potential adverse effects could occur due to disproportionate heating, and facilitates optimized selection of treatment parameters to achieve improved efficiency in selective thermo-mechanical lysis interventions.

According to a preferred embodiment of the first aspect of the invention, the sensing surface of the transducer (51) is planar and/or adapted to the shape of the layer of ink (21) or melanin (22) and/or to the shape of the surface (3) of the skin tissue.

It is noted that, in some embodiments, a generally planar configuration of the transducer (51) sensing surface is sufficient to provide accurate optoacoustic measurements, due to the relatively small surface area of the transducer compared to typical body curvatures. Even though human or animal skin surfaces (3) may have a general curvature, the small dimensions of the sensing surface of the transducer (51)-for instance, in the sub-millimiter, millimiter or centimetre scale, in diameter or length-allow for an effective approximation of the skin surface (3) as planar during measurement. This planar approximation ensures that optoacoustic wavefronts arrive substantially in phase at the transducer (51), minimizing destructive interference and thus increasing measurement precision and signal clarity. In some other embodiments, the sensing surface of the transducer (51) may be slightly curved, being slightly concave or convexe, accounting for part, an average or certain body curvatures.

It is further noted that, according to some embodiments, the transducer (51) may have a sensing surface specifically adapted or shaped to conform closely to more complex anatomical regions, including areas exhibiting pronounced curvature or irregular shapes such as elbows, knees, shoulders, fingers, toes, ankles, feet, or facial features. For such embodiments, the sensing surface of the transducer (51) may present a concave, convex, or otherwise curved profile, designed to substantially match or approximate the local curvature of the skin surface (3).

According to some embodiments, the device (1) may further comprise interchangeable or removable measurement heads or modules, each containing a dedicated transducer (51) with sensing surfaces of different shapes and sizes. Such interchangeable heads may allow for flexibility in adapting the device (1) to varying anatomical locations and specific measurement conditions, ensuring consistent and accurate measurements regardless of patient anatomy or positioning requirements. Examples of interchangeable heads include modules having planar surfaces for generally flat skin regions, curved surfaces with varying radii of curvature for limbs and joints, and specialized shapes tailored to smaller or more delicate anatomical regions.

It is noted that, in some embodiments of the invention, the ultrasonic detector (5) may comprise a piezoelectric crystal arranged at the sensing surface of the transducer (51), wherein said crystal is preferably shaped to substantially follow the geometric distribution of the tattoo ink granules within the skin. Typically, the tattoo ink granules approximate a slab-like geometry whose upper and lower boundaries lie substantially parallel to the skin surface (3). Consequently, the piezoelectric crystal is preferably configured in a corresponding planar shape, such as a disc-shaped, rectangular, square, or other substantially planar configurations, as previously commented. Under these circumstances, optoacoustic wavefronts originating from the ink layer (21) can arrive substantially in phase at the piezoelectric crystal, leading to constructive interference and generating a strong and distinct electrical signal upon conversion from mechanical vibrations. This significantly enhances the amplitude and clarity of the detected tattoo-specific signal relative to other high-absorbing skin constituents, such as blood vessels or non-targeted tissues.

It is further noted that, in some preferred embodiments, alternative transducer technologies such as capacitive micromachined ultrasound transducers (CMUTs) or optical-based ultrasound detection techniques may be employed instead of traditional piezoelectric crystals. CMUTs, for example, offer advantageous performance characteristics such as improved sensitivity, wide frequency bandwidth, ease of integration into compact measurement heads, and compatibility with advanced electronic interfaces. Similarly, optical-based ultrasound detection methods, including interferometric or optical resonator-based approaches, provide non-contact detection and exhibit high sensitivity, enabling accurate capture of subtle optoacoustic wave variations with minimal mechanical coupling requirements.

It is additionally noted that, analogous to the tattoo ink layer (21), the melanin-containing layer (22) typically exhibits slab-like geometry but may generally be thinner than the ink layer. Accordingly, a planar or appropriately adapted transducer shape also facilitates in-phase arrival of the optoacoustic waves generated within this melanin layer (22). In instances involving skin tissue with substantial melanin concentration, the optoacoustic signal detected by a planar transducer (51) will characteristically comprise two temporally distinct components: an initial component attributable to the melanin layer (22), followed by a later component corresponding to the deeper tattoo ink layer (21). The processing unit (6) in preferred embodiments is thus configured to calculate the envelope of these signals, employing mathematical techniques such as the Hilbert transform or similar operations, and subsequently generate and display a three-dimensional image clearly distinguishing and representing heat deposition within both the melanin-containing layer (22) and the tattoo ink layer (21).

Advantageously, by providing a transducer (51) sensing surface specifically adapted to the anatomical characteristics of the skin surface (3) or the underlying targeted layers (21, 22), the device (1) improves the consistency, accuracy, and reliability of optoacoustic wave detection, allowing the waves to be collected in phase and avoiding negative interferences. This tailored adaptation significantly reduces measurement errors arising from wavefront distortions or phase cancellations, thus enhancing the predictive accuracy of thermo-mechanical lysis outcomes and enabling precise optimization of treatment parameters across diverse anatomical sites.

According to a preferred embodiment of the first aspect of the invention, the device (1) further comprises or is in communication with a display configured to display the data and/or the image comprising information related to the deposited heat of the one or more target layers (21, 22).

It is noted that, in some embodiments of the invention, the display may comprise a dedicated integrated visual output component forming an integral part of the device (1), such as an LCD, LED, OLED, AMOLED, or e-ink display panel, providing immediate and direct visualization of measurement results. Alternatively, the device (1) may communicate wirelessly or through wired connections (such as HDMI, USB, Ethernet, or DisplayPort) with external displays, including conventional monitors, touchscreen tablets, smartphones, augmented reality devices, or projection-based systems, thus offering flexibility and convenience tailored to user preferences or operational environments. The displayed data may include numeric values, textual information, graphs, or detailed visualizations like heatmaps or 2D or 3D reconstructions representing the deposited heat within targeted layers (21, 22).

It is further noted that, in preferred embodiments, the display may support interactive user interfaces enabling real-time manipulation of the visualized data. Examples of such interactive capabilities include zooming, rotating, slicing through 3D images, adjusting visualization thresholds, selecting specific target layers (e.g., tattoo ink, melanin), or modifying displayed parameters. Additionally, the interface may provide comparative views, allowing simultaneous visualization of reference data alongside measurement results, facilitating rapid assessment and informed decision-making during pre-treatment evaluations. The graphical interface may also comprise indicators or alerts highlighting critical or abnormal heat deposition levels to immediately inform practitioners of potential risks or inefficiencies.

Advantageously, by incorporating a dedicated display or enabling communication with external visualization systems, the device (1) significantly provides clear and immediate feedback regarding optoacoustic measurements. This visual clarity allows practitioners to intuitively interpret complex heat deposition data, ensuring informed and accurate predictions of the outcomes of selective thermo-mechanical lysis procedures, thereby improving treatment efficiency, patient safety, and overall operational confidence.

According to a preferred embodiment of the first aspect of the invention, the memory comprises further instructions which, when executed by the processing unit (6), cause said processing unit (6) to generate a heatmap (8) representing the amount of heat deposited in one or more target layers (21, 22).

It is noted that, in some embodiments of the invention, the heatmap (8) generated by the processing unit (6) may visually represent the spatial distribution and intensity of heat deposition within target layers (21, 22), such as the tattoo ink layer (21) and/or melanin-containing layer (22). The heatmap may use color-coded representations-such as ranging from cool colors (e.g., blue or green) indicating low heat deposition, to warm colors (e.g., yellow, orange, or red) indicating high heat deposition-although grayscale gradients or other visual coding schemes may also be employed. Preferably uses a grayscale. Additionally, the heatmap may be presented as two-dimensional cross-sectional images, three-dimensional volumetric renderings, or as layered representations allowing selective viewing of specific target layers individually or collectively.

It is further noted that, according to some embodiments, the heatmap (8) may be interactive, enabling practitioners to manipulate visualizations directly through an interface connected to the display. Interactive functionalities may include adjusting thresholds for highlighting critical heat levels, zooming and rotating three-dimensional visualizations, comparing heatmaps from different measurement sessions or conditions, or overlaying the heatmap onto photographic or schematic images of the skin area being analyzed. Such heatmap data may also be exported digitally for external analysis, stored electronically in patient records, or integrated into comprehensive treatment-planning software for subsequent selective thermo-mechanical lysis procedures.

Advantageously, by providing a visually intuitive heatmap (8), the device (1) offers practitioners an effective tool for rapidly assessing heat deposition distribution and intensity within target skin layers (21, 22). This immediate visual insight facilitates accurate interpretation and informed decision-making, directly contributing to optimized parameter selection, improved procedural efficacy, and enhanced safety during selective thermo-mechanical lysis treatments.

According to a preferred embodiment of the first aspect of the invention, the memory comprises further instructions which, when executed by the processing unit (6), cause said processing unit (6) to select a wavelength optimized for heat deposition in one or more target layers (21, 22), preferably by comparing absorbed heat quantities for different wavelengths of the electromagnetic radiation pulses.

It is noted that, in some embodiments of the invention, the processing unit (6) may systematically analyse and compare the heat deposition data obtained from optoacoustic measurements at multiple wavelengths, which may typically range from 400 nm to 1200 nm. Preferably, such analysis involves emitting radiation pulses at a series of predetermined wavelengths selected based on known absorption spectra of common tattoo pigments or melanin, thus enabling accurate identification of optimal wavelengths. Alternative embodiments may dynamically select and test wavelengths across broader spectral regions, possibly utilizing tuneable laser sources, optical parametric oscillators (OPOs), or multiple discrete wavelength sources, to experimentally determine the wavelength providing the highest targeted heat deposition efficiency.

It is further noted that the wavelength optimization performed by the processing unit (6) may consider various factors such as differential absorption between multiple ink colours, pigment densities, or depths, as well as variations in melanin concentration. By evaluating comparative heat absorption characteristics at distinct wavelengths, the processing unit (6) may identify wavelengths specifically tailored to maximize selective heat deposition in the desired target layer (21, 22), while minimizing unwanted heat absorption in surrounding tissues. Such analysis may be presented visually through graphical interfaces, numeric tables, comparative heatmaps, or other intuitive formats facilitating practitioner interpretation and selection of treatment parameters.

Advantageously, the wavelength selection capability provided by the device (1) substantially enhances the precision and effectiveness of selective thermo-mechanical lysis treatments. By accurately identifying and selecting optimal wavelengths tailored to specific patient characteristics or treatment objectives, the invention ensures improved procedural efficiency, reduced risk of adverse effects, and optimized outcomes in tattoo removal, skin whitening, or melanin-containing spot removal procedures.

According to a preferred embodiment of the first aspect of the invention, the memory comprises further instructions which, when executed by the processing unit (6), cause said processing unit (6) to select a wavelength that optimizes a ratio of deposited heat in the ink layer (21) with respect to deposited heat in the melanin layer (22), preferably by analysing, for different wavelengths of the electromagnetic radiation pulses, the detected optoacoustic waves (70, 71) to extract the quantity of deposited heat for each wavelength at the ink layer (21) and at the melanin-containing layer (22), and comparing the ratios of deposited heat by the melanin-containing layer (22) and the ink layer (21).

It is noted that, in some embodiments of the invention, the processing unit (6) evaluates optoacoustic signals generated at multiple test wavelengths to precisely determine the differential absorption characteristics of the tattoo ink and melanin-containing layers. Such evaluation may involve sequentially emitting electromagnetic pulses at various wavelengths, for example, wavelengths specifically selected within a range of approximately 400 nm to 1200 nm, including but not limited to common wavelengths like 532 nm, 694 nm, 755 nm, 810 nm, or 1064 nm. By quantifying the absorbed heat separately for each wavelength and each targeted layer, the processing unit (6) can calculate the heat deposition ratio, identifying wavelengths that provide maximum selective absorption in the tattoo ink layer (21), while minimizing unwanted heating of the melanin-containing layer (22).

It is further noted that the selection of optimal wavelengths by the processing unit (6) may account for different tattoo colours, pigment types, melanin concentrations, and skin phototypes. In certain embodiments, multiple ratios may be calculated, for example by considering the deposited heat across several pigment or melanin layers simultaneously. Additionally, the device (1) may present this information via numeric indicators, graphical plots, heatmaps, or comparative charts, facilitating immediate and intuitive interpretation by practitioners, and enabling dynamic adjustment of laser parameters to match individual patient conditions.

Advantageously, by optimizing the heat deposition ratio between the tattoo ink layer (21) and the melanin-containing layer (22), the device (1) ensures highly selective and safe application of subsequent thermo-mechanical lysis treatments. This wavelength optimization significantly reduces risks associated with excessive heating of melanin-rich tissues, such as burns, whitening or hypopigmentation, while effectively maximizing the treatment efficacy for targeted tattoo or pigment removal procedures.

According to a preferred embodiment of the first aspect of the invention, the device (1) further comprises means to measure the distance between the sensing surface of the transducer (51) and the surface (3) of the skin tissue, preferably wherein said means are the ultrasonic detector (5) configured to operate in ultrasound reflection mode.

It is noted that, in some embodiments, the ultrasonic detector (5) itself may advantageously serve as a dual-function component, performing both optoacoustic wave detection and distance measurement by operating in a reflection mode. In such configurations, the transducer (51) may emit short, high-frequency ultrasound pulses directed toward the surface (3) of the skin, and subsequently detect the reflected echoes to calculate the precise distance between the sensing surface and the skin surface (3), based on the known speed of sound propagation in air, coupling media, or tissues. Alternatively, in some embodiments the device (1) may include other suitable means for distance measurement, such as laser distance sensors, infrared proximity sensors, capacitive distance sensors, or mechanical distance gauges integrated within or positioned externally to the measurement head. The determination of this distance allows precise interpretation of optoacoustic signals, improving measurement accuracy, consistency in positioning during sequential measurements, and aiding automated device operation by guiding practitioners in maintaining optimal and reproducible placement of the transducer (51).

Advantageously, by incorporating distance measurement means, such as by utilizing the ultrasonic detector (5) in ultrasound reflection mode, the device (1) can detect the distance to the surface of the skin, which may be employed to fine-tune parameters of the radiation source, among other uses.

According to a preferred embodiment of the first aspect of the invention, the memory comprises further instructions which, when executed by the processing unit (6), cause said processing unit (6) to calculate the distance from the sensing surface of the transducer (51) to a heat-absorbing material (91) preferably placed on the surface (3) of the skin tissue, wherein the heat-absorbing material (91) may be the same or different than the reference element, by analysing the optoacoustic wave generated in the heat-absorbing material (91) when irradiated with the radiation source.

It is noted that, in some embodiments, the heat-absorbing material (91) is preferably positioned directly on the surface (3) of the skin, thus providing a precise and consistent reference point for calculating distances. Suitable heat-absorbing materials (91) include, but are not limited to, thin carbon-based films or threads, dyed polymeric films, metal foils (e.g., gold, aluminum), or specialized gels or pads containing highly absorptive pigments. Upon irradiation by pulses from the radiation source (4), this material generates a distinct optoacoustic wave, detectable by the transducer (51). The processing unit (6) then calculates the distance from the sensing surface of the transducer (51) to the skin surface (3) based on the known speed of sound propagation and the time-of-flight (TOF) between radiation emission and the detected optoacoustic wave arrival. Such configurations allow for accurate, reproducible, and rapid calibration of device positioning relative to the skin surface, thereby significantly enhancing measurement precision. Preferably, the heat-absorbing material (91) is a black or dark thread, fibre or cord, more preferably it is flexible. In some embodiments it is a flexible black or dark layer.

Advantageously, by precisely determining the distance between the transducer (51) and skin surface (3) using a heat-absorbing material (91), the device (1) allows to select adequate parameters, such as laser spot size, that guarantee the effectiveness and safety of selective thermo-mechanical lysis treatments.

According to a preferred embodiment of the first aspect of the invention, the device (1) further comprises a removable piece (9), wherein the removable piece (9) comprises the heat-absorbing material (91) and/or the reference element, such that when the removable piece (9) is attached to the device (1) and the device (1) is applied on the surface (3) of the skin tissue for optoacoustic measurement, the heat-absorbing material (91) and/or the reference element rests on the skin of said biological material, preferably wherein the heat-absorbing material (91) is a black thread.

It is noted that, in some embodiments, the removable piece (9) may have diverse configurations designed for easy attachment and detachment from the device (1), facilitating rapid calibration or positioning steps. For instance, the removable piece (9) may comprise holder, ring, clip, adhesive patch, or rigid frame designed to attach to the device (1) and to securely position the heat-absorbing material (91) or reference element against the skin surface (3). The heat-absorbing material (91) is preferably implemented as a black thread or filament made from materials such as carbon fiber, dyed nylon, silk, polyester, or similar highly absorbent thread. Alternative forms for the heat-absorbing material include thin carbon sheets, pigmented flexible films, or metallic foils integrated within the removable piece (9).

Advantageously, the provision of a removable piece (9) carrying the heat-absorbing material (91) significantly enhances operational convenience and consistency, allowing practitioners to rapidly perform precise distance calibration and optoacoustic signal referencing, thereby directly contributing to more accurate, reliable, and effective selective thermo-mechanical lysis interventions.

According to a preferred embodiment of the first aspect of the invention, the memory comprises further instructions which, when executed by the processing unit (6), cause said processing unit (6) to determine an optimal laser spot size for heat deposition in one or more of the target layers (21, 22), as a function of the distance between the one or more target layers (21, 22) and the detector surface of the transducer (51), and/or the distance between the surface (3) of the skin tissue and one or more of the target layers.

It is noted that, in some embodiments, the optimal laser spot size calculated by the processing unit (6) depends on the depth or relative position of the targeted layer within the skin. Generally, larger spot sizes may be advantageously selected for deeper target layers (21, 22) inside the skin tissue, and/or those positioned further away from the transducer surface, ensuring sufficient energy penetration and uniform heat deposition at the desired depth. Conversely, smaller spot sizes might be selected for superficial layers or layers closer to the transducer surface, thus optimizing localized heating without unnecessarily affecting surrounding tissues. The processing unit (6) preferably calculates the optimal spot size based on measured distances, preferably obtained via any of the previously described distance-measurement techniques (such as ultrasound reflection, time-of-flight analyses), and may dynamically adapt spot size recommendations accordingly. Alternative embodiments may further consider additional parameters such as pigment density, melanin concentration, tissue type, and optical properties to refine spot size optimization.

Advantageously, the automatic determination of optimal laser spot size as a function of the target layer's depth ensures precise and effective heat deposition, directly contributing to safer, more efficient, and tailored selective thermo-mechanical lysis treatments, significantly reducing risks of collateral tissue damage and enhancing procedural success rates.

According to a preferred embodiment of the first aspect of the invention, the device (1) further comprises a housing that encases at least the ultrasonic detector (5), the housing surrounding the sensing surface of the transducer (51) of the detector with walls extending outwardly, preferably between 1 and 10 mm in height, the encasing further comprising one or more apertures (53) in said walls suitable to allow excess gel to exit upon application of the gel and the distal end of said walls to the skin surface (3), thereby preventing bubble formation in between the sensing surface of the transducer (51) and the surface (3) of the skin tissue.

It is noted that, in some embodiments, the housing surrounding the ultrasonic detector (5) may have walls configured with varying heights within the specified range (e.g., 1 mm, 3 mm, 5 mm, 8 mm, or 10 mm or more), suitably adapted to different anatomical regions or device sizes. The apertures (53) provided in said walls may have diverse shapes and configurations, such as circular, elliptical, slit-shaped, rectangular, or irregular openings, designed to efficiently channel excess acoustic coupling gel out of the enclosed area when pressure is applied to the skin surface (3). This arrangement prevents air entrapment and bubble formation, which can otherwise impair optoacoustic signal detection by causing acoustic impedance mismatches. Additionally, the apertures (53) may be positioned symmetrically or asymmetrically around the transducer (51) surface to optimize gel removal regardless of the orientation of the device (1), further enhancing usability and consistency during measurement procedures.

Advantageously, by incorporating a housing with apertures (53) specifically designed for avoiding air entrapment, bubble formation and allowing effective gel displacement, the device (1) ensures optimal acoustic coupling and significantly improves the reliability and accuracy of optoacoustic measurements, avoiding reflections or surface effects due to the presence of bubbles that introduce a different medium of transmission with a different sound velocity.

According to a preferred embodiment of the first aspect of the invention, the memory comprises further instructions which, when executed by the processing unit (6), cause said processing unit (6) to filter noise in the detected optoacoustic waves (70, 71) by applying a noise filtering algorithm based on a Discrete Fourier Transform (DFT) algorithm.

It is noted that, in some embodiments, the processing unit (6) utilizes a Discrete Fourier Transform-based noise filtering approach to effectively isolate meaningful optoacoustic signals from background noise and interference. The noise-filtering algorithm may involve transforming the detected optoacoustic waves (70, 71) into the frequency domain, selectively suppressing frequency components associated with noise or unwanted interferences, and subsequently reconstructing a cleaned signal through inverse transformation. Alternative embodiments may apply variations such as Fast Fourier Transform (FFT), band-pass filtering, notch filtering, or adaptive frequency-domain filtering techniques. The parameters of the filtering process, such as frequency cutoffs or suppression thresholds, may be dynamically adapted based on real-time analysis of signal quality, the type of skin tissue, the presence of artifacts, or device-specific calibration characteristics, thereby optimizing the clarity and reliability of the extracted heat-deposition information.

Advantageously, applying a DFT-based noise filtering algorithm significantly improves the quality and precision of optoacoustic measurements, enabling more accurate heat-deposition assessments and thus directly contributing to safer, more reliable, and more effective selective thermo-mechanical lysis procedures.

According to a preferred embodiment of the invention, the memory comprises further instructions which, when executed by the processing unit (6) cause said processing unit (6) to filter noise in the detected optoacustic waves (70, 71) by applying a noise filtering algorithm based on the following mathematical transform: ${C}_{{d}_{l}} = {\sum }_{n = - \left(M-1\right) / 2}^{\left(M-1\right) / 2} P \left({d}_{n}\right) {φ}_{{d}_{l}} \left({d}_{n}\right)$

Wherein *C_{dₗ}* are the coefficients of the components of the optoacoustic wave signal *P*(*dₙ*) expressed as a function of the distance to the detector *dₙ*, and the wave components of the signal are *φ_{dₗ}*(*dₙ*) wherein *φ_{dₗ}*(*dₙ*) can be expressed as: ${φ}_{{d}_{l}} \left({d}_{n}\right) = \left\{\begin{matrix}\frac{- 1}{\sqrt{2}} if {d}_{n} = {d}_{l} \\ \frac{1}{\sqrt{2}} if - {d}_{n} = {d}_{l} \\ 0 otherwise\end{matrix}\right.$

**Wherein** *dₗ* is the diameter of the wave emitter.

One of the main problems that optoacoustic system face is that the signals are corrupted with noise, coming from different sources. For example, electrical fast fluctuations inherent to the analog circuitry of the imaging system usually adds random high frequency noise to the signals. There are also parts of the equipment, generally elements of the ultrasound detector (e.g. acoustic lenses or acoustic matching materials) that add low frequency noise with a lower degree of randomness.

It is, therefore, convenient to remove the effects of such noise in the signals of the invented device.

The discrete Fourier transforms plays a preponderant role for this essential signal conditioning procedures. The strength of the Fourier transform for signal processing is rooted in the fact that it can be used to decompose a given discrete signal into a sum of sinusoidal functions whose terms can be manipulated at will. Each of these sine and cosine functions has a specific frequency and its contribution to the summation is weighted by a coefficient. The lower frequency sinusoidals carry coarse detail information of the signal while the higher frequencies carry the finer. By filtering the coefficients, the contribution to the signal of each sinusoidal component can be weighted as desired. Like this, specific features of the signal can be enhanced or attenuated while preserving the remainder. The coefficients, known as Fourier coefficients, are obtained by applying the Discrete Fourier Transform to the original signal.

Similarly, the discrete wavelet transform decomposes each signal into of a sum of functions which can be then modulated at will by changing the value of their respective coefficients. Like in the Fourier case, each function is related to the coarser or finer details of the signal, however, they can also be shifted in time.

In both the discrete wavelet transform and the discrete Fourier transform, the functional components are called the basis functions, and must fulfil several mathematical properties. More specifically, they should form a Hamel basis. For the discrete wavelet transform there is large variety of "basis families" each of them with different form but fulfilling the mentioned mathematical properties.

However, both the discrete Fourier transform and discrete wavelet transform were derived as "general purpose algorithms" and are used across all technologies.

In this document we disclose a transform (namely the Discrete Opto-Acoustic Transform, DOAT), inspired on the physics of optoacoustic signal generation that has important advantages over classical transforms that arise from the fact that it resembles the physics of optoacoustic signal formation. First, it breaks through the computational complexity of the discrete fourier transform and the discrete wavelet transform. Second, it has a better performance for essential optoacoustic signal processing operations related to low frequency and high frequency noise removal.

The DOAT can be described as follows: let us assume that we have an optoacoustic signal P with M elements each of them corresponding to a discrete time point *tₙ , P*(*tₙ*) = (*p*_{*t*₁},*p*_{*t*₂}, ... ... ,*p_{t_{M}}*), where M is uneven. If we multiply the time values by the speed of sound we obtain the signal as a function of the distance to the detector *d'ₙ*. Moreover, by making a change of variables, using the central point of the signal ((M-1)/2 + 1 ) as a reference, we can express the signal as *P*(*dₙ*) *=* (*p_{d}*-_{(_{*M*-1})/2}, ... , *p*-_{*d*₂}, *p*-_{*d*₁}, *p*_{*d*₀}, *p*_{*d*₁}, *p*_{*d*₂}, ... , *p*_{*d*(_{*M*-1)/2}} ). Where *d*₀ = 0 and *P*(*dₙ*) = *P*(*tₙ*). We can now define a set of DOAT basis functions *φ_{dₗ}*(*dₙ*) each of them having the same length as *P*(*dₙ*) *.*

The number of functions contained in the set is (M-1)/2 and each function is characterized by the value of *dₗ* as: ${φ}_{{d}_{l}} \left({d}_{n}\right) = \left\{\begin{matrix}\frac{- 1}{\sqrt{2}} if {d}_{n} = {d}_{l} \\ \frac{1}{\sqrt{2}} if - {d}_{n} = {d}_{l} \\ 0 otherwise\end{matrix}\right.$

The values of *dₗ* range from *d*₁ to *d*_{(*M*-1)/2} . In order to obtain the DOAT coefficients, *C_{dₗ}* , one must apply the following formula (the DOAT transform): ${C}_{{d}_{l}} = {\sum }_{n = - \left(M-1\right) / 2}^{\left(M-1\right) / 2} P \left({d}_{n}\right) {φ}_{{d}_{l}} \left({d}_{n}\right)$

To obtain the original signal from the DOAT coefficients and basis functions we apply: $P \left({d}_{n}\right) = {\sum }_{l = 1}^{\frac{M - 1}{2}} {C}_{{d}_{l}} {φ}_{{d}_{l}} \left({d}_{n}\right)$

Like the Fourier transform or the wavelet transform, the basis functions are orthonormal and the coefficients *C_{dₗ}* can be filtered for signal conditioning and other operations. The advantage of the DOAT transform for optoacoustic signals is that the DOAT takes into consideration the physics of optoacoustic signal generation and therefore it is better suited for signal conditioning retrieving better the signal compromised by noise.

The optoacoustic transform is related to physics of optoacoustic signal generation as follows: The signal emitted from a sphere has a characteristic N shape, containing information on the diameter of the sphere (which is the reciprocal distance between the vertical post of the N shape and the amount of light absorbed (which is the peak-to-peak distance of the N). However, to build the N function one only needs the position of the posts and height of such post. The DOAT basis functions correspond to the vertical post of such N while the coefficients correspond to the amount of light absorbed by the sphere. *dₗ* corresponds to the diameter of the each sphere and *C_{dₗ}* the contribution of a sphere of radius *dₗ* to the signal.

In a preferred embodiment of the invention the raw optoacoustic signals are processed using the discrete optoacoustic transform as follows: Firstly the DOAT transform is calculated. Then the resulting coefficients are filtered, then the inverse DOAT transform is calculated from the filtered coefficients. If the filters are selected correctly the high frequency and low frequency noise is removed from the signal.

In a further preferred embodiment of the invention the processing unit is configured to filter the coefficients *C_{dₗ}* using a moving average.

In a another preferred embodiment of the invention the processing unit is configured to filter the coefficients *C_{dₗ}* setting to zero all the coefficients below zero,

Yet in a further preferred embodiment of the invention the processing unit is configured to filter the coefficients *C_{dₗ}* setting to zero all the coefficient above a certain sphere radius (*dₗ*)

The DOAT transform has to be modified to be applied in for signals that are the result of the combination of different objects and whose center cannot be localized obtaining the SDOAT which can be expressed as follows: ${C}_{d} = \sum P ⋅ {w}_{l , m} ⋅ {φ}_{d}$

Where *w*_{*l*,*m*} is the window function, with length *l* and shifted m positions around the origin.

In a preferred embodiment of the invention the processing unit is configured to process the raw optoacoustic signals using the SDOAT and then, for each window, the processing unit is configured to filter the coefficients and calculate the inverse DOAT, obtaining the filtered signal.

The Fourier transform can be used for implementing filters as a function of the size of the objects to be imaged, i.e the object that emit the signals. In such operation, the optoacoustic signals are filtered in frequency bands which are then reconstructed separately displaying different objects.

In a preferred embodiment of the invention the processing unit is configure band pass filters using the Fourier transform are applied and images are form for each bandpass. In a preferred method, the amount of ink granules of a given size is calculated from the intensity frequency bands.

The SDOAT can be used to filter out the signal depending on the size of the optoacoustic emitters. The SDOAT performance for this task is more direct that the fourier transform, since the SDOAT coefficients are directly related to the radius of a spherical source.

Advantageously, employing the DOAT or SDOAT to extract the relevant optoacoustic waves allows filtering with unparalleled precision most of the noise and obtaining a clean profile for the optoacoustic wave, as they are transforms tailored for this precise purpose.

According to a preferred embodiment of the first aspect of the invention, the memory comprises further instructions which, when executed by the processing unit (6), cause said processing unit (6) to apply a Hilbert transform to the noise-filtered optoacoustic waves to extract the signal envelope (72, 73) and obtaining the heat deposition profile from said signal envelope (72, 73).

It is noted that the extraction of the signal envelope using a Hilbert transform is particularly beneficial since the envelope directly represents the instantaneous amplitude of the optoacoustic signal, which closely correlates with the spatial and temporal distribution of deposited heat. Alternative mathematical operations, such as wavelet transforms, squared-envelope methods, or short-time Fourier transforms (STFT), may also be employed to extract similar amplitude profiles. Moreover, the heat deposition profile obtained from the envelope may in some embodiments be visualized in multiple useful formats, including graphical plots, heatmaps, cross-sectional images, or three-dimensional reconstructions, providing flexible tools for analysis tailored to user needs or preferences. In certain embodiments, the envelope may further undergo smoothing or interpolation techniques, such as Savitzky-Golay filters, polynomial fitting, or spline interpolation, to enhance the clarity and continuity of the heat deposition profile, thereby improving the precision of layer characterization. Additionally, by analyzing peak amplitudes, widths, or integrated areas under the envelope curves, the processing unit (6) may quantify parameters such as total deposited energy, relative heat distribution, or layer thicknesses, enabling detailed comparisons between multiple measurements, different skin conditions, or varying treatment parameters.

Advantageously, utilizing the Hilbert transform to extract the signal envelope significantly improves clarity and interpretability of the heat deposition profile, thereby enhancing predictive accuracy and enabling optimized, targeted, and safe selective thermo-mechanical lysis treatments.

According to a preferred embodiment of the first aspect of the invention, the processing unit (6) is configured to differentiate optoacoustic waves (70, 71) corresponding to one or more target layers (21, 22) from those corresponding to surrounding tissue elements, preferably by applying a thresholding algorithm based on a predetermined percentage of the maximum signal amplitude.

It is noted that the thresholding algorithm employed in some embodiments by the processing unit (6) may allow effective isolation of signals originating from targeted structures, such as tattoo inks or melanin-rich layers, from background signals produced by surrounding tissues, including blood vessels, dermal components, or other non-targeted absorptive elements. Preferably, the threshold percentage may be selected within a range, for example between 10% and 70%, more typically around 25%, to ensure optimal discrimination based on the relative intensity of the optoacoustic signals. In alternative embodiments, the threshold may be dynamically adjusted depending on individual patient characteristics, skin phototypes, or pigment densities, and it may also be established by comparison with reference signals generated by predefined high-absorption calibration materials. Other embodiments may incorporate additional classification techniques, such as adaptive thresholding, signal-to-noise ratio (SNR)-based methods, or machine-learning algorithms to further enhance differentiation accuracy, particularly in complex tissue environments or in cases involving multiple overlapping target layers.

Advantageously, employing threshold-based differentiation significantly improves the clarity, reliability, and precision of identifying target layers, directly contributing to safer, more accurate, and effective outcomes in selective thermo-mechanical lysis interventions.

According to a preferred embodiment of the first aspect of the invention, the radiation source (4) can also operate as a thermo-mechanical lysis system, or the device (1) further comprises, or is in communication with, a thermo-mechanical lysis system.

It is noted that, in some embodiments, the radiation source (4) itself may be configured to directly perform selective thermo-mechanical lysis treatments, effectively integrating both measurement and treatment functions into a single unit. Examples of radiation sources suitable for combined measurement and treatment include high-energy pulsed lasers such as Q-switched lasers, picosecond lasers, femtosecond lasers, or nanosecond lasers emitting radiation typically between 400 nm and 1200 nm. Alternatively, the device (1) may communicate or interface with external, separate thermo-mechanical lysis systems through wired or wireless connections, allowing treatment parameters (wavelength, energy, pulse duration, and beam diameter) optimized by the processing unit (6) to be transmitted and directly applied in subsequent treatments. This integration may encompass automated synchronization, data-sharing capabilities, or manual parameter entry, facilitating efficient workflows and tailored adjustments of treatment conditions based on real-time optoacoustic measurements.

Advantageously, integrating or communicating directly with a thermo-mechanical lysis system enables seamless transition from precise measurement to optimized treatment, significantly enhancing procedure safety, accuracy, and clinical efficiency.

According to a preferred embodiment of the first aspect of the invention, the memory comprises further instructions which, when executed by the processing unit (6), cause said processing unit (6) to calculate a probability estimate of the success of a thermo-mechanical lysis non-therapeutic procedure, based on the deposited heat in at least one of the target layers (21, 22), wherein said probability is preferably calculated for at least two wavelengths of the radiation source (4).

It is noted that the probability estimate calculation performed by the processing unit (6) in some embodiments involves analysing deposited heat values obtained at multiple wavelengths, preferably comparing results from two or more wavelengths selected based on pigment absorption characteristics, such as 532 nm, 694 nm, 755 nm, or 1064 nm. In some embodiments the processing unit (6) may determine optimal wavelengths by identifying the one providing maximum heat deposition specifically within the ink layer (21), while simultaneously minimizing undesired absorption in the melanin-containing layer (22). Moreover, the device may factor in tattoo depth information derived from the previously generated optoacoustic images, selecting optimal beam diameters, wherein larger beam diameters may be preferred for deeper tattoos.

Further, the probability calculations may integrate additional data such as pigment density, skin phototype, historical patient response to treatments, or predefined clinical thresholds. Alternative embodiments may employ machine-learning algorithms trained on clinical data to improve predictive accuracy. Visualization of success probabilities could be provided numerically or graphically, enhancing clinical decision-making prior to the initiation of actual treatments.

Advantageously, calculating probability estimates based on measured heat deposition at different wavelengths significantly improves treatment predictability, ensures optimal parameter selection, and thus directly enhances the safety and effectiveness of selective thermo-mechanical lysis procedures.

According to a preferred embodiment of the first aspect of the invention, the processing unit (6) is further configured to calculate a probability estimate of the success of a thermo-mechanical lysis non-therapeutic procedure, based on the comparison of the deposited heat in the ink layer (21) and the deposited heat in the melanin layer (22) and/or in the reference element.

It is noted that this comparative probability estimation in some embodiments involves detailed analysis of heat absorption ratios or relative heat deposition between the ink and melanin-containing layers, or between the ink layer and a reference element. Specifically, the processing unit (6) may evaluate whether melanin absorption significantly competes with or diminishes effective heating of the tattoo ink, if the objective is to remove the tattoo, potentially indicating lower treatment success or increased risk of side effects. In cases where the melanin-containing layer absorbs excessive unwanted radiation, the procedure could become ineffective for ink clearance, or lead to adverse outcomes such as burns, scarring, or undesired skin whitening. For example, in cases where a whitening is the objective, good heat deposition in the melamine layer is sought. The processing unit (6) may implement specific thresholds or ratio limits that indicate successful or problematic outcomes, providing clear guidance for laser parameter adjustments. Furthermore, alternative analysis methods, such as multi-factorial scoring systems, threshold-based decision algorithms, or machine-learning models trained on historical patient data, could be employed to enhance predictive accuracy. Results of this analysis could be visually or numerically displayed, highlighting clearly whether parameters should be modified or whether the treatment should be reconsidered or avoided altogether.

Advantageously, this comparative analysis of deposited heat between ink and melanin layers significantly reduces risk, allowing practitioners to precisely optimize laser parameters, thus ensuring safer and more effective thermo-mechanical lysis outcomes.

According to a preferred embodiment of the first aspect of the invention, the memory comprises further instructions which, when executed by the processing unit (6), cause said processing unit (6) to estimate granule size in the ink layer (21) and/or in the melanin-containing layer (22) from optoacoustic wave analysis, preferably before and after a thermo-mechanical lysis non-therapeutic procedure.

It is noted that granule size estimation using optoacoustic wave analysis involves may involve analysing frequency spectra, amplitude profiles, or temporal characteristics of the detected waves. Specific properties of optoacoustic signals, like local maximum and minimum values, frequency shifts or spectral broadening, may correlate closely with granule size changes caused by thermo-mechanical lysis, facilitating quantifiable assessments of treatment-induced granule fragmentation or removal effectiveness. The processing unit (6) may apply in some embodiments various analytical techniques, including spectral peak identification, spectral centroid methods, or machine-learning classification algorithms trained on known granule-size reference data. Furthermore, it is noted that comparative granule-size analysis before and after procedures provides direct insight into treatment effectiveness, allowing dynamic adjustments in subsequent sessions, such as modifying pulse energy, pulse duration, or wavelength to optimize fragmentation and clearance. Alternative embodiments may also integrate additional imaging modalities, such as microscopy reference images or in-vivo imaging techniques, to cross-validate or calibrate optoacoustic-based granule size estimations. Results may be visually presented via graphs, images, numerical data, or comparative tables, clearly indicating granule size distributions and changes occurring during treatments.

Advantageously, accurate granule-size estimation significantly improves treatment evaluation, directly enabling precise optimization of parameters and enhancing the overall effectiveness and reliability of selective thermo-mechanical lysis procedures.

It is noted that many practitioners perform the so-called "R2O" method, which consist in doing multiple laser passes the same day instead of waiting for weeks to perform a new pass. To this end the practitioners waits for ~20 minutes after the first pass, until the whitening is gone. The "white frost" prevents the laser light to reach the tattoo due to increased photon scattering. When whitening is gone, a new pass can be performed. However, the practitioner has to rely on his visual perception of whitening to decide whether a new pass can be made or not, which is highly subjective.

It is important to note that the invention enables the possibility of using the device to calculate the amount of heat deposited in the tattoo, retrieved by the processing unit, after performing a high energy laser pass corresponding to a Thermo-Mechanical Lysis (TML) device. In this situation, the skin presents a white frost that fades away with time which, in turn, does not allow to perform another TML laser pass. The white frost highly scatters the photons, not allowing the laser light to reach the tattoo. As explained above, in many occasions the practitioners wait until the white frost fades away to perform another laser pass during the same session instead of waiting several weeks (the R20 method). By using the invention, the practitioner can have a precise estimation of the amount of light that will be absorbed by the tattoo not needing to rely on a visual observation of the "whiteness" of the area to be threated. Like this the practitioner may perform the additional high energy laser passes earlier, saving session time. In a similar manner, the invention can be used to assess the positive effect that skin patches (for example, silicone infused with perfluorodecalin) may have on the whitening effect, reducing the amount of scattering.

A method utilizing the device may comprise, for example, estimating how much heat will be deposited in a target layer with the device and/or method of the invention, performing an initial high-energy laser pass using a thermo-mechanical lysis device to treat the target layer, and deciding, based on the previous estimation, if another one or more passes will be needed, even if there was been white frost creation that prevents to evaluate the state of the target later.

According to a second aspect of the invention a non-therapeutic use of a device (1) according to any one of the previous claims is disclosed. Said non-threapeutic use related to predicting the outcome of a thermo-mechanical lysis treatment and/or estimate skin damage or whitening associated with said thermo-mechanical lysis treatment, preferably wherein the thermo-mechanical lysis treatment is used for tattoo removal or for the removal of skin lentigines, melasma or other dark spots related to aging

It is noted that the in some embodiments, the non-therapeutic use of the device (1) encompasses employing optoacoustic measurements to anticipate both the efficacy and potential adverse effects of selective thermo-mechanical lysis treatments, particularly for aesthetic or cosmetic interventions such as tattoo removal, skin whitening, or the reduction of skin lentigines, melasma, age spots, and other hyperpigmented lesions. The prediction of treatment outcomes may involve analysing parameters such as the intensity, distribution, and ratios of deposited heat within targeted skin layers, utilizing, for example, imaging and analytical capabilities previously described. In some embodiments, the prediction may include comparative evaluations at multiple wavelengths to identify optimal treatment settings, thereby avoiding ineffective or unsafe laser parameters. Additionally, the non-therapeutic use may incorporate detailed analyses of melanin-related absorption to estimate risks of collateral skin damage, burns, or undesired whitening, facilitating personalized assessments tailored to diverse skin types, tones, and conditions. Alternative embodiments may involve integrating historical patient data, advanced statistical methods, or machine-learning models trained to recognize subtle indicators of successful or problematic outcomes, thereby enhancing predictive accuracy and clinical utility.

Advantageously, the disclosed non-therapeutic use allows practitioners to reliably forecast aesthetic treatment outcomes, significantly enhancing patient safety and treatment effectiveness by proactively identifying and mitigating risks associated with selective thermo-mechanical lysis interventions.

According to a third embodiment of the invention, a non-therapeutic method is disclosed. IT is noted that the non-therapeutic method of the invention allows determining the relative deposited heat between an ink layer (21) comprised in skin tissue, a melanin-containing layer (22) comprised in said skin tissue, and/or a reference element of a high light absorption material not comprised in said skin tissue, wherein the method comprises:
I. irradiating the skin tissue with pulsed electromagnetic from a radiation source (4);
II. detecting waves (70, 71) generated by the interaction of the radiation with the ink layer (21), the melanin-containing layer, and/or the reference element, wherein the detecting is made with an ultrasonic detector (5) comprising a transducer (51) with a sensing surface, wherein the sensing surface is planar or has the shape of the layer of ink (21) or melanin (22) and/or the shape of the surface (3) of the skin tissue;
III. analysing with a processing unit (6) the detected optoacoustic waves (70, 71) to determine the relative difference in absorbed power between any two of the ink layer (21), the melanin-containing layer (22) and the reference element;
IV. preferably, displaying on a display data and/or an image comprising information related to the relative difference in absorbed power.

In a preferred embodiment of the invention, the method further comprises generating a heat map (8) representing the energy absorbed by any combination of the ink layer (21), the melanin-containing layer and the reference element.

In a preferred embodiment of the invention, the method further comprises determining the distance between the transducer (51) sensing surface of the ultrasonic detector (5) and the ink layer (21) and/or melanin-containing layer, preferably comprising displaying said distance or an image representation of said distance in the display.

In a preferred embodiment of the invention, the method further comprises determining the thickness (210, 220) of the melanin-containing layer (22) and/or the ink layer (21) and, preferably, determining their relative distance, and more preferably displaying said thicknesses (210, 220) and/or said relative distance in the display.

n a preferred embodiment of the invention, the method further comprises:
V. obtaining optoacoustic waves (70, 71) at two or more wavelengths;
VI. determining the most suitable laser wavelength from the two or more wavelengths for a thermo-mechanical lysis treatment based on the ratio of heat deposited in any at least two layers of the melanin-containing layer (22), the ink layer (21) and the reference element, and/or based solely on the deposited heat the ink layer (21).

In a preferred embodiment of the invention, the method further comprises determining the optimal laser spot size as a function of the distance between the transducer (51) sensing surface of the ultrasonic detector (5) and the ink layer (21) or the melanin-containing layer.

In a preferred embodiment of the invention, the method further comprises filtering noise in the detected optoacustic waves by applying a noise filtering algorithm based on a Discrete Fourier Transform (DFT) algorithm.

In a preferred embodiment of the invention, the method further comprises filtering noise in the detected optoacustic waves (70, 71) by applying a noise filtering algorithm based on the following mathematical transform: ${C}_{{d}_{l}} = {\sum }_{n = - \left(M-1\right) / 2}^{\left(M-1\right) / 2} P \left({d}_{n}\right) {φ}_{{d}_{l}} \left({d}_{n}\right)$

Wherein *C_{dₗ}* are the coefficients of the components of an the signal *P*(*dₙ*) expressed as a function of the distance to the detector *dₙ*, and the wave componennts of the signal are *φ_{dₗ}*(*dₙ*) wherein *φ_{dₗ}*(*dₙ*) can be expressed as: ${φ}_{{d}_{l}} \left({d}_{n}\right) = \left\{\begin{matrix}\frac{- 1}{\sqrt{2}} if {d}_{n} = {d}_{l} \\ \frac{1}{\sqrt{2}} if - {d}_{n} = {d}_{l} \\ 0 otherwise\end{matrix}\right.$

Wherein *dₗ* is the diameter of the wave emitter.

In a preferred embodiment of the invention, the method further comprises applying a Hilbert transform to the noise-filtered optoacustic waves to extract the signal envelope (72, 73) and obtaining an energy absorption profile from said signal envelope.

In a preferred embodiment of the invention, the method further comprises calculating a probability estimate of the success of a non-therapeutic thermo-mechanical lysis procedure, based on the relative deposited heat in the ink layer (21) and the melanin-containing layer (22), preferably the method further comprising displaying said probability estimate of the success of a laser treatment procedure and/or an image comprising visual information related to said probability estimate of the success of a laser treatment procedure.

In a preferred embodiment of the invention, the method further comprises:
VII. estimating granule size in the composition of the ink layer (21) and/or in the melanin-containing layer (22) before a thermo-mechanical lysis non-therapeutic procedure;
VII. estimating granule size in the composition of the ink layer (21) and/or in the melanin-containing layer (22) after a thermo-mechanical lysis non-therapeutic procedure; and comparing the granule size estimated before and after the laser non-therapeutic procedure and determine based on said comparison if the procedure was successful.

To enable a better understanding of the present disclosure, reference will now be made, by way of example only, to the accompanying schematic drawings. It is important to note that the following schematic drawings are provided solely as exemplary, non-limiting potential embodiments of the invention. Accordingly, the drawings are to be regarded as illustrative instead of restrictive, serving merely to aid in the explanation of the invention's principles and potential applications.

Figure 1 illustrates a schematic transverse cross-section of a non-limiting example of a device (1) according to one or more embodiments of the invention, configured for optoacoustic measurement of deposited heat in target layers (21, 22). In particular, the device (1) is depicted positioned above a surface (3) of skin tissue, which includes at least one ink layer (21) and a melanin-containing layer (22). The ink layer (21) may correspond, for example, to a tattooed region, whereas the melanin-containing layer (22) represents a naturally pigmented superficial layer of skin.

In Figure 1, the device (1) comprises a radiation source (4), illustrated as a "LASER HEAD," configured to emit pulsed electromagnetic radiation. The radiation source (4) may be, as non-limiting examples, a Q-switched laser, diode laser, picosecond or femtosecond pulsed laser, or other suitable sources with wavelengths typically between 400 nm and 1200 nm. The emitted laser radiation is guided through an optical fibre or optical delivery means (41) that directs the pulses precisely toward the surface (3) of the skin. It is noted that optical fibre may alternatively be replaced by free-space optical arrangements, articulated arms, or fibre bundles, depending on specific application requirements or device ergonomics.

Furthermore, the ultrasonic detector (5), located adjacent to the optical delivery means (41), comprises a transducer (51) configured to detect the optoacoustic waves generated by the interaction of the emitted radiation with the target layers (21, 22). The transducer (51) may be implemented using materials such as piezoelectric ceramics (e.g., PZT), piezo-polymers (PVDF), or capacitive micromachined ultrasonic transducers (CMUTs). The sensing surface of the transducer (51) shown is planar, but could alternatively exhibit curvature or adapt its shape according to different anatomical regions or targeted layer geometries, as previously described.

Additionally, the ultrasonic detector (5) is connected to a processing unit (6), illustrated here as comprising an analog-to-digital converter (ADC) and a graphics processing unit (GPU), which performs the analysis of the received optoacoustic signals. It is noted that the processing unit (6) may alternatively comprise digital signal processors (DSPs), field-programmable gate arrays (FPGAs), microcontrollers, or general-purpose CPUs in various configurations, and/or comprise or be connected to a controller. The GPU facilitates rapid and efficient computation, image reconstruction, signal analysis, and visualization tasks. Alternative embodiments may incorporate cloud-based memories, processing units or remote analysis systems, enhancing flexibility and scalability in clinical or research environments. Additionally, the device (1) may be employed woth coupling materials such as acoustic gels or matching layers positioned between the transducer (51) and skin surface (3) to enhance acoustic impedance matching and signal quality.

Figure 2 illustrates alternative embodiments of the device (1), highlighting certain aspects not previously detailed, with some previously described elements omitted for clarity and illustrative purposes. Nevertheless, it is noted that in practical embodiments, the device (1) preferably still comprises other components such as an electromagnetic radiation source (4), processing unit (6), and/or any necessary controllers or electronic components.

In particular, Figure 2(a) introduces one or more apertures (53) integrated into the housing encasing the ultrasonic detector (5). These apertures (53) serve to effectively expel air and excess acoustic coupling gel when the device (1) is pressed against the surface (3) of the skin, thus preventing bubble formation and ensuring reliable acoustic coupling between the sensing surface of the transducer (51) and the skin surface (3). The apertures (53) may vary in size, shape, and quantity; for instance, they could be circular, rectangular, slit-shaped, or arranged symmetrically or asymmetrically to optimize gel expulsion depending on device orientation and specific use-cases. This illustration lacks the melanin-containing layer (22), which is intentionally omitted to simplify the visualization of key structural relationships in this specific embodiment, or that may be too thin to significantly affect measurement outcomes.

Figure 2(b) provides a bottom view depicting a spatial relationship between the sensing surface of the transducer (51), indicated by the darkened central region (52), and the underlying tattoo ink layer (21). It is noted that the sensing surface of the transducer (51) may exhibit varying dimensions, typically ranging from one or more millimeters to a few centimeters in diameter, to suitably match the size of the targeted tattooed region (21) and enhance optoacoustic signal detection efficiency.

Figure 3 schematically illustrates several analytical steps involved in processing optoacoustic signals captured by the transducer (51), specifically relating to the measurement of heat deposition in a tattoo ink layer (21). Panel (a) shows a simplified geometric representation of the transducer (51), with length (510), positioned above a tattoo ink layer (21) characterized by a specific thickness (210). Dashed lines (7) schematically represent the propagation of optoacoustic wavefronts generated by thermoelastic expansion upon irradiation by electromagnetic pulses. The dimensions shown (510, 210) are exemplary, and actual thicknesses and lengths may vary widely depending on anatomical location, type of tattoo, or clinical conditions.

Panel (b) illustrates a simplified representation of a typical optoacoustic wave (70) detected by the transducer (51). This wave exhibits an "N-shaped" profile, where the amplitude (height) of the signal correlates to the magnitude of deposited heat, and the temporal distance between vertical segments relates directly to the thickness (210) of the tattoo slab (21). In practice, optoacoustic signals (70) are considerably noisier and more complex. Thus, the simplified waveform shown here presumes prior noise reduction via sophisticated filtering techniques such as Discrete Optoacoustic Transform (DOAT) or Sparse DOAT (SDOAT), involving transformation to the frequency or another suitable domain, selective noise reduction, and subsequent inverse transformation to yield a clearer, analysable waveform.

In panels (c) and (d), the detected optoacoustic wave (70) is converted from the time domain to a spatial representation by multiplying time-of-flight values by the acoustic propagation velocity within the skin (generally between about 1450 m/s to 1650 m/s) and optoacoustic gel, which preferably matches that one of the skin. This conversion allows precise determination of spatial parameters, such as the thickness (210) of the tattoo ink slab (21), from temporal features of the detected waveform. Panel (e) illustrates the extraction of a signal envelope (72) from the spatially converted waveform, utilizing analytical methods such as the Hilbert transform, wavelet transforms, or comparable envelope extraction algorithms. This envelope (72) clearly delineates the spatial distribution and intensity of the heat deposition profile.

Finally, panel (f) shows a schematic heatmap (8) that visually represents the measured heat deposition within the tattoo ink layer (21), corresponding to its physical dimensions (510, 210) or a representation of them that may not be to scale. Such heatmaps (8) preferably employ colour or grayscale gradients to quantitatively display regions of differing heat intensity, thus providing intuitive visualization and facilitating rapid assessment of optoacoustic measurement outcomes. Alternative visualizations may include three-dimensional reconstructions, cross-sectional representations, or numerical data representations.

Figure 4 presents similar analytical steps as previously described in Figure 3 but additionally introduces a melanin-containing layer (22) positioned superficially above the tattoo ink layer (21). Panel (a) indicates both the melanin layer thickness (220) and the tattoo ink layer thickness (210), along with the transducer length (510). Panels (b), (c), and (d) illustrate optoacoustic waves (70, 71) corresponding respectively to the tattoo ink layer (21) and melanin-containing layer (22). Each layer generates a distinct optoacoustic wave, where the temporal and spatial separation between these signals allows clear differentiation of the layers. Panel (e) shows two distinct envelopes (72, 73), derived through methods such as the Hilbert transform, individually representing deposited heat profiles for each of these layers. Finally, panel (f) provides a schematic heatmap (8) displaying separate heat deposition profiles for both the tattoo ink and melanin layers, facilitating direct comparative analysis and optimized parameter selection to reduce the risk of unintended melanin heating and related side effects during thermo-mechanical lysis procedures.

Figures 5 and 6 illustrate schematic representations of the removable piece (9), which includes a heat-absorbing material (91), which may be employed for distance measurement, calibration, and/or optoacoustic signal referencing in the device (1). Specifically, Figure 5 depicts multiple views of the removable piece (9): perspective view (a), bottom view (b), and side view (c). The heat-absorbing material (91) is depicted here as a flexible black thread or filament, which may be fabricated from materials such as carbon fibre, silk, nylon, polyester, or other suitable threads with high optical absorption characteristics. Alternative embodiments of the heat-absorbing material (91) might include thin carbon films, pigmented layers, or metallic foils integrated into the removable piece (9).

Figure 6 illustrates how the removable piece (9) may be positioned and integrated with the distal end of the device (1). Specifically, Figure 6(a) shows a schematic cross-section, clearly illustrating the removable piece (9) attached at the distal end, encasing the sensing surface of the transducer (51), thus positioning the heat-absorbing material directly in front of the ultrasonic detector (5). Figure 8(b) shows an external side view of the assembled device (1), indicating how the removable piece (9) seamlessly integrates into the overall device design. Alternative designs for the removable piece (9) could involve different attachment mechanisms, such as snap-fit, magnetic coupling, friction fit, or threaded connectors, facilitating rapid and secure positioning during measurement preparations or calibration processes.

It is noted that the figures provided are merely representational and may not be drawn to scale. Certain proportions thereof may be exaggerated, while others may be minimized. The figures are intended to illustrate various implementations of the invention that can be understood and appropriately carried out by those of ordinary skill in the art. Commonly designated elements among the various figures refer to common or equivalent elements in the depicted embodiments. The figures are not intended to be exhaustive or to limit the invention to the precise form disclosed. It should be understood that the invention can be practiced with modification and alteration, and that the invention be limited only by the claims and the equivalents thereof.

## Claims

1. A device (1) for optoacoustic measurement of deposited heat in one or more target layers (21, 22) comprised in human or animal skin tissue, wherein the device (1) comprises:
a. a radiation source (4) configured to emit pulsed electromagnetic radiation suitable for generating optoacoustic waves (70, 71);
b. an ultrasonic detector (5) for detecting optoacoustic waves (70, 71) generated by the interaction of the emitted radiation with the one or more target layers (21, 22) (2), the ultrasonic detector (5) comprising a transducer (51) with a sensing surface; and
c. a processing unit (6) in communication with the radiation source (4) and the ultrasonic detector (5), and in communication with a memory;
**characterized in that** the memory comprises instructions which, when executed by the processing unit (6) cause said processing unit (6) to:
i. determine the distance between the sensing surface of the transducer (51) and one or more of the target layers (21, 22) from the relationship between the time of the radiation emission, the optoacoustic wave received as a function of time and the sound velocity;
ii. optionally, if it is detected optoacoustic waves (70, 71) associated to more than one target layer (21, 22), determine an indication of the relative distance between two or more of said target layers (21, 22) by analysing the time difference of said optoacoustic waves (70, 71);
iii. determine the thickness of one or more target layers (21, 22), by analysing the detected optoacoustic waves (70, 71) associated to target layers (21, 22)said target layers (21, 22); and
iv. generate data and/or an image comprising information related to the deposited heat in the one or more target layers (21, 22), their distance to the sensing surface of the transducer (51) and/or their respective thickness.
wherein the one or more target layers (21, 22) comprise at least an ink layer (21) and/or a melanin layer (22).

2. The device (1) according to claim 1, configured to be calibrated by measuring a reference optoacoustic wave from a reference element of a high light absorption coefficient material, preferably wherein the optoacoustic waves (70, 71) detected in step (b) are normalized or expressed in percentage compared with the reference wave, more preferably wherein the reference element is a reference layer.

3. The device (1) according to any one of the previous claims, wherein the memory comprises further instructions which, when executed by the processing unit (6) cause said processing unit (6) to:
v. analyse the detected optoacoustic waves (70, 71) to determine the relative difference in deposited heat between any two of the ink layer (21), the melanin-containing layer and the reference element, or between the three of them;
vi. generate data and/or an image comprising information related to the relative difference in deposited heat calculated in step (iii)

4. The device (1) according to any one of the previous claims, wherein the memory comprises further instructions which, when executed by the processing unit (6) cause said processing unit (6) to generate a heatmap (8) representing the amount of heat deposited in one or more target layers (21, 22).

5. The device (1) according to any one of the previous claims, wherein the memory comprises further instructions which, when executed by the processing unit (6) cause said processing unit (6) to select a wavelength that optimizes a ratio of deposited heat in the ink layer (21) with respect to deposited heat in the melanin layer (22), by analysing, for different wavelengths of the electromagnetic radiation pulses, the detected optoacoustic waves (70, 71) to extract the quantity of deposited heat for each wavelength at the ink layer (21) and at the melanin-containing layer (22), and comparing the ratios of deposited heat by the melanin-containing layer (22) and the ink layer (21).

6. The device (1) according to any one of the previous claims, further comprising a removable piece (9), wherein the removable piece (9) comprises the heat-absorbing material (91) and/or the reference element, such that when the removable piece (9) is attached to the device (1) and the device (1) is applied on the surface (3) of the skin tissue for optoacoustic measurement, the heat-absorbing material (9) and/or the reference element rests on the skin of said biological material, preferably wherein the heat-absorbing material (9) is a black thread.

7. The device (1) according to any one of the previous claims, wherein the memory comprises further instructions which, when executed by the processing unit (6) cause said processing unit (6) to filter noise in the detected optoacustic waves (70, 71) by applying a noise filtering algorithm based on the following mathematical transform: ${C}_{{d}_{l}} = {\sum }_{n = - \left(M-1\right) / 2}^{\left(M-1\right) / 2} P \left({d}_{n}\right) {φ}_{{d}_{l}} \left({d}_{n}\right)$ Wherein *C_{dₗ}* are the coefficients of the components of the optoacoustic wave signal *P*(*dₙ*) expressed as a function of the distance to the detector *dₙ*, and the wave components of the signal are *φ_{dₗ}*(*dₙ*) wherein *φ_{dₗ}*(*dₙ*) can be expressed as: ${φ}_{{d}_{l}} \left({d}_{n}\right) = \left\{\begin{matrix}\frac{- 1}{\sqrt{2}} if {d}_{n} = {d}_{l} \\ \frac{1}{\sqrt{2}} if - {d}_{n} = {d}_{l} \\ 0 otherwise\end{matrix}\right.$ Wherein *dₗ* is the diameter of the wave emitter.

8. The device (1) according to any one of the previous claims, wherein the memory comprises further instructions which, when executed by the processing unit (6) cause said processing unit (6) to calculate a probability estimate of the success of a thermo-mechanical lysis non-therapeutic procedure, based on the deposited heat in at least one of the target layers (21, 22), wherein said probability is preferably calculated for at least two wavelengths of the radiation source (4), and/or based on the comparison of the deposited heat in the ink layer (21) and the deposited heat in the melanin layer (22) and/or in the reference element.

9. The device (1) according to any one of the previous claims, wherein the memory comprises further instructions which, when executed by the processing unit (6) cause said processing unit (6) to estimate granule size in the ink layer (21) and/or in the melanin-containing layer (22) from optoacoustic wave analysis, preferably before and after a thermo-mechanical lysis non-therapeutic procedure.

10. Non-therapeutic use of a device (1) according to any one of the previous claims to predict the outcome of a thermo-mechanical lysis treatment and/or estimate skin damage or whitening associated with said thermo-mechanical lysis treatment, preferably wherein the thermo-mechanical lysis treatment is used for tattoo removal or for the removal of skin lentigines, melasma or other dark spots related to aging.

11. A non-therapeutic method for determining the relative deposited heat between an ink layer (21) comprised in skin tissue, a melanin-containing layer (22) comprised in said skin tissue, and/or a reference element of a high light absorption material not comprised in said skin tissue, the method comprising:
(I) irradiating the skin tissue with pulsed electromagnetic from a radiation source (4);
(II) detecting waves (70, 71) generated by the interaction of the radiation with the ink layer (21), the melanin-containing layer, and/or the reference element, wherein the detecting is made with an ultrasonic detector (5) comprising a transducer (51) with a sensing surface, wherein the sensing surface is planar or has the shape of the layer of ink (21) or melanin (22) and/or the shape of the surface (3) of the skin tissue;
(III) analysing with a processing unit (6) the detected optoacoustic waves (70, 71) to determine the relative difference in absorbed power between any two of the ink layer (21), the melanin-containing layer (22) and the reference element;
(IV) preferably, displaying on a display data and/or an image comprising information related to the relative difference in absorbed power, preferably wherein said image is a heat map (8) representing the energy absorbed by any combination of the ink layer (21), the melanin-containing layer and the reference element.

12. The method according to any claim 11, further comprising:
(V) obtaining optoacoustic waves (70, 71) at two or more wavelengths;
(VI) determining the most suitable laser wavelength from the two or more wavelengths for a thermo-mechanical lysis treatment based on the ratio of heat deposited in any at least two layers of the melanin-containing layer (22), the ink layer (21) and the reference element, and/or based solely on the deposited heat the ink layer (21).

13. The method according to any one of claims 11 to 12, further comprising filtering noise in the detected optoacustic waves (70, 71) by applying a noise filtering algorithm based on the following mathematical transform: ${C}_{{d}_{l}} = {\sum }_{n = - \left(M-1\right) / 2}^{\left(M-1\right) / 2} P \left({d}_{n}\right) {φ}_{{d}_{l}} \left({d}_{n}\right)$ Wherein *C_{dₗ}* are the coefficients of the components of an the signal *P*(*dₙ*) expressed as a function of the distance to the detector *dₙ*, and the wave componennts of the signal are *φ_{dₗ}*(*dₙ*) wherein *φ_{dₗ}*(*dₙ*) can be expressed as: ${φ}_{{d}_{l}} \left({d}_{n}\right) = \left\{\begin{matrix}\frac{- 1}{\sqrt{2}} if {d}_{n} = {d}_{l} \\ \frac{1}{\sqrt{2}} if - {d}_{n} = {d}_{l} \\ 0 otherwise\end{matrix}\right.$ Wherein *dₗ* is the diameter of the wave emitter.

14. The method according to any one of claims 11 to 13, further comprising calculating a probability estimate of the success of a non-therapeutic thermo-mechanical lysis procedure, based on the relative deposited heat in the ink layer (21) and the melanin-containing layer (22), preferably the method further comprising displaying said probability estimate of the success of a laser treatment procedure and/or an image comprising visual information related to said probability estimate of the success of a laser treatment procedure.

15. The method according to any one of claims 11 to 14, further comprising:
(VII) estimating granule size in the composition of the ink layer (21) and/or in the melanin-containing layer (22) before a thermo-mechanical lysis non-therapeutic procedure;
(VIII) estimating granule size in the composition of the ink layer (21) and/or in the melanin-containing layer (22) after a thermo-mechanical lysis non-therapeutic procedure; and
(IX) comparing the granule size estimated before and after the laser non-therapeutic procedure and determine based on said comparison if the procedure was successful.
